# EUROPÄISCHE PATENTSCHRIFT

(11) **EP 2 315 738 B1**
(45) Veröffentlichungstag und Bekanntmachung des Hinweises auf die Patenterteilung: **02.09.2015**
(21) Anmeldenummer: 09781221.8
(22) Anmeldetag: 29.07.2009
(51) Int. Cl.: C07C 45/35, C07C 51/25, C07C 5/333, C07C 57/055, B01J 35/04, B01J 23/888, B01J 23/00, B01J 19/24, B01J 37/02, B01J 8/06

(54) **EINSATZ VON SCHAUMKÖRPERN IN OXIDATIONS-REAKTOREN ZUR HERSTELLUNG UNGESÄTTIGTER ALDEHYDE ODER CARBONSÄUREN**
USE OF FOAM BODIES IN OXIDATION REACTORS FOR PREPARING UNSATURATED ALDEHYDES OR CARBOXYLIC ACIDS
UTILISATION DE CORPS EN MOUSSE DANS DES RÉACTEURS À OXYDATION POUR LA PRÉPARATION D'ALDÉHYDES OU D'ACIDES CARBOXYLIQUES INSATURÉS

(30) Priorität: 29.08.2008 DE 102008044946
(43) Veröffentlichungstag der Anmeldung: 04.05.2011
(73) Patentinhaber: Evonik Degussa GmbH, 45128 Essen (DE)
(72) Erfinder: ZANTHOFF, Horst-Werner, 45481 Mülheim an der Ruhr (DE); BRAUSCH, Nicole, 45657 Recklinghausen (DE); KUPPINGER, Franz-Felix, 45768 Marl (DE); SAUER, Jörg, 48249 Dülmen (DE); SABBAGH, Andreas, 64625 Bensheim (DE); FRÜHLING, Dennis, 44805 Bochum (DE); WEBER, Markus, 46240Bottrop (DE); BECKER, Oliver, 45772 Marl (DE)
(86) Internationale Anmeldenummer: PCT/EP2009/059782
(87) Internationale Veröffentlichungsnummer: WO 2010/023053

(56) Entgegenhaltungen:
- EP-A1- 1 916 230
- EP-A1- 1 935 868
- US-A1- 2004 220 434
- US-A1- 2005 239 643

## Beschreibung

Die vorliegende Erfindung betrifft ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen sowie ein Verfahren zur Herstellung eines auf einer ungesättigten Carbonsäure basierenden Polymers.

Acrylsäure ist als ein Partialoxidationsprodukt des Propens ein bedeutendes Monomer, das als solches zur Herstellung superabsorbierender Polymerisate oder in Form seiner Alkylester zur Erzeugung von beispielsweise Klebstoffen Verwendung findet (vgl. z. B. WO-A-02/055469 und WO-A-03/078378). Acrolein ist ein bedeutendes Zwischenprodukt, beispielsweise für die Herstellung von Glutardialdehyd, Methionin, Folsäure und Acrylsäure.

Methacrylsäure und Methacrylsäureester, wie Methylmethacrylat und Butylinethacrylat, werden ebenfalls in einer großen Anwendungsvielfalt verwendet. Typische Endanwendungen beinhalten Acrylkunststoffbahnen bzw. -folien, Preßharze, Polyvinylchlorid-Modifizierungsmittel, Verarbeitungshilfsstoffe, Acryllacke, Fußbodenpflegemittel, Versiegelungs- bzw. Dichtmittel, Autogetriebeflüssigkeiten, Kurbelgehäuseöl-Modifizierungsmittel, Kraftfahrzeugbeschichtungen, Ionenaustauscherharze, Zement- bzw. klebstoffmodifizierungsmittel, Wasserbehandlungspolymere, elektronische Haftmittel, Metallbeschichtungen und Acrylfasern.

Die bekannten, großtechnischen Verfahren zur Herstellung von (Meth)acrolein und/oder (Meth)acrylsäure (beispielsweise nach DE-A-19 62 431) durch Gasphasenoxidation von Propen bzw. Isobuten werden in der Regel in Rohrbündelreaktoren durchgeführt, die eine große Anzahl (teilweise mehr als 30.000) zwischen Rohrböden eingeschweißte Reaktionsrohre aufweisen. Vorzugsweise wird hierbei bei Temperaturen zwischen 200 und 450°C und gegebenenfalls erhöhtem Druck gearbeitet. Die Reaktionsrohre sind mit dem heterogenen Oxidations-Katalysator in Form einer Festbettschüttung gefällt und werden vom Reaktionsgemisch durchströmt. Bei den Festbettschüttungen handelt es sich vorzugsweise um zu Kugeln, Ringen oder Zylindern geformte, auf Metallmischoxiden basierende Katalysatormassen oder aber um sogenannte Schalenkatalysatoren, die durch das Beschichten von vorgeformten, inerten Trägerkörpern mit einer katalytisch aktiven Masse erhalten wurden.

EP 1935868 A1 beschreibt ein Verfahren zur Herstellung von ungesättigten Carboxylsäuren und ungesättigten Nitrilen aus den korrespondierenden C 3 bis C 5 Alkanen oder Mischungen von C 3 bis C 5 Alkanen und Alkenen, enthaltend eine Oxidation in Gegenwart eines geträgerten Mo-V basierten Mischmetalloxidkata-lysators in einem Mehrstufenreaktionssystem, welches eine Separation des Oxidationsproduktes von einem oder mehrerer der Zwischenproduktströme sowie eine Zugabe von weiterem Sauerstoff in die Reaktionszonen, die der ersten Reaktionszone folgen.

EP 1916230 A1 offenbart einen integrierten Multizonenprozess zur Umwandlung von Alkanen zu den korrespondierenden Alkenen, welcher eine exotherme Umwandlung eines Teils der Alkane zu den entsprechenden Alkenen durch oxidative Dehydrogenierung in einer exothermen Reaktionszone in Gegenwart von Sauerstoff und einem geeigneten Katalysator und ein Einleiten des Produktes der exothemren Reaktionszone in eine endotherme Reaktionszone, in der zumindest ein Teil der verbleibenden nicht umgewandelten Alkane in Gegenwart von Kohlendioxid und einem anderen geeigneten Katalysator endothermisch dehydrogeniert wird, umfasst.

US 2004/220434 A1 beschreibt ein Verfahren zur Umwandlung eines Kohlenwasserstoffreaktanden in ein Produkt enthaltend ein Oxygenat oder ein Ntril, bei dem (A) eine Reaktandenzusammensetzung enthaltend Kohlenwasserstoffreaktant, Sauerstoff oder eine Sauerstoffquelle und optional Ammoniak in einen Reaktor, der Mikrokanäle und einen Katalysator aufweist, geleitet wird und dort eine exotherme Reaktion stattfindet, und das Reaktionsprodukt, (B) die Wärme aus dem Mikrokanalreaktor während Schritt (A) in einen transferiert wird und (C) das Produkt aus Schritt (A) gequencht wird.

US 2005/239643 A1 beschreibt strukturierte Katalysatoren, die zur Oxidation von Alkanen, Alkenen und Mischungen davon geeignet sind, wobei die Katalysatoren ein oder mehrere Mischmetalloxidkatalysatoren und eine dreidimensionale, selbsttragende Struktur aufweisen, und die Struktur der Katalysatoren die Bewegung von gasförmigen Reaktanden und Produkten erleichtert.

Die Zielsetzung einer jeden zweistufigen Festbettgasphasenoxidation von Propen zu Acrylsäure bzw. von Isobuten zu Methacrylsäure oder auch einer Umsetzung von Propan bzw. Isobutan zu Acrylsäure bzw. Methacrylsäure über eine Dehydrierung, gefolgt von einer zweistufigen Festbettgasphasenoxidation oder aber über eine Direktoxidation des Propans bzw. Isobutans besteht grundsätzlich darin, eine möglichst hohe Raum-Zeit-Ausbeute an Acrylsäure bzw. Methacrylsäure (RZA_{As} bzw. RZA_{MAS}) zu erzielen (das ist bei einer kontinuierlichen Verfahrensweise die je Stunde und Gesamtvolumen der verwendeten Katalysatorschüttung in Litern erzeugte Gesamtmenge an Acrylsäure bzw. an Methacrylsäure). Es besteht deshalb generelles Interesse daran, eine solche zweistufige Festbettgasphasenoxidation von Propen zu Acrylsäure bzw. von Isobuten zu Methacrylsäure einerseits unter einer möglichst hohen Belastung der ersten Festbettkatalysatorschüttung mit Propen bzw. Isobuten (darunter wird die Menge an Propen bzw. Isobuten in Normlitern (= N1; das Volumen in Liter, das die entsprechende Propen- bzw. Isobuten-Menge bei 25°C und 1 bar, einnehmen würde) verstanden, die als Bestandteil des Reaktionsgasausgangsgemisches pro Stunde durch einen Liter an Katalysatorschüttung geführt wird) und andererseits unter einer möglichst hohen Belastung der zweiten Festbettkatalysatorschüttung mit Acrolein bzw. Methacrolein (darunter wird die Menge an Acrolein bzw. Methacrolein in Normlitern (= N1, das Volumen in Liter, das die entsprechende Acrolein- bzw. Methacrolein-Menge bei 25°C und 1 bar, einnehmen würde) verstanden, die als Bestandteil des Reaktionsgasausgangsgemisches pro Stunde durch einen Liter an Katalysatorschüttung geführt wird) durchzuführen, ohne dabei den beim einmaligen Durchgang des Reaktionsgasausgangsgemisches durch die beiden Festbettkatalysatorschüttungen erfolgenden Umsatz an Propen bzw. Isobuten und Acrolein bzw. Methacrolein sowie die über beide Reaktionsstufen bilanzierte Selektivität der damit einhergehenden Acryl- bzw. Methacrylsäure-Bildung (bezogen auf umgesetztes Propen bzw. Isobuten) nennenswert zu beeinträchtigen.

Die Realisierung des Vorgenannten wird durch die Tatsache beeinträchtigt, daß sowohl die Festbettgasphasenoxidation von Propen bzw. Isobuten zu Acrolein bzw. Methacrolein als auch die Festbettgasphasenoxidation von Acrolein bzw. Methacrolein zu Acrylsäure bzw. Methacrylsäure einerseits stark exotherm verläuft und andererseits von einer Vielfalt möglicher Parallel- und Folgereaktionen begleitet wird. Mit zunehmender Propen- bzw. Isobuten-Belastung bzw. mit zunehmender Acrolein- bzw. Methacrolein-Belastung der jeweiligen Festbettkatalysatorschüttung muss, bei Verwirklichung der angestrebten Randbedingung eines im Wesentlichen gleichbleibenden Propen- bzw. Isobuten-Umsatzes und eines im Wesentlichen gleichbleibenden Acrolein- bzw. Methacrolein-Umsatzes, daher davon ausgegangen werden, daß infolge der erhöhten Wärmeproduktion die Selektivität der Wertproduktbildung abnimmt (siehe zum Beispiel auch EP-A-450 596).

Um die Selektivität der Wertproduktbildung zu verbessern, ist es daher erforderlich, die bei der Umsetzung des Propens bzw. des Isobutents sowie die bei der Umsetzung des Acroleins bzw. Methacroleins freiwerdende Reaktionswärme effektiv abzuführen. In den eingangs beschriebenen Rohrbündelreaktoren wird daher auf der Mantelseite des Rohrbündelreaktors um die Reaktionsrohre wenigstens ein Wärmeaustauschmittel geführt, bei dem es sich beispielsweise um eine Salzschmelze handelt.

Die vorstehend beschriebenen Rohrbündelreaktoren des Standes der Technik weisen jedoch zahlreiche Nachteile auf.

Zum einen kann trotz des Einsatzes von Wärmeaustauschmitteln die freigesetzte Reaktionswärme in den Katalysatorfestbettschüttungen nur begrenzt abgeführt werden, was insbesondere auf die schlechte Wärmeabfuhr der Festbettschüttungen selbst zurückzuführen ist. Diese schlechte Wärmeabfuhr der Festbettschüttungen limitiert derzeit die Rohrdurchmesser auf ca. 2,5 bis 3 cm, was den Einsatz von teilweise mehr als 30.000_Reaktionsrohren erforderlich macht, um eine zufriedenstellende Raum-Zeit-Ausbeute sicherzustellen. Diese hohe Zahl an Reaktionsrohren bedingt jedoch aufgrund der großen Zahl an Schweißverbindungen hohe Investitionskosten. Auch bedingt diese hohe Anzahl an Reaktionsrohren sehr komplexe Katalysator-Füllprozeduren, bei denen sichergestellt werden muss, dass in jedem der Reaktionsrohre die gleiche Katalysatormenge und eine vergleichbare Schüttdichte eingebracht wird. Dieses ist wichtig, da anderenfalls der Druckverlust nicht in allen Reaktionsrohren gleich ist. Ein gleichmäßiger Druckverlust in den Reaktionsrohren ist mitentscheidend für sie Sicherstellung eines gleichmäßigen Eduktumsatzes und die Minimierung der Nebenproduktbildung.

Weiterhin kommt es trotz des Einsatzes des Wärmeabführmittels auch bei Rohrdurchmessern von lediglich 2,5 bis 3 cm noch immer zur Ausbildung eines Temperaturprofil von teilweise mehr als 90°C in axialer Richtung des Reaktors. Dabei kommt es in den Reaktionsrohren zu sogenannten "*Hot-Spots*" (das sind Bereiche mit besonders hoher Wärmeentwicklung), welche insbesondere auch die Lebensdauer der Katalysatoren in diesen Bereichen zeitlich beschränkt. Die Differenz zwischen der Temperatur des Wärmeaustauschmittels und des Katalysators im *Hot-Spot* nimmt mit zunehmendem Durchsatz zu. Auch kann die Bildung dieser Heisspunkttemperaturen zu einer Abnahme der Selektivität bei der Produktumsetzung führen. Zur Verbesserung der Selektivität, mit der die Zielprodukte gebildet werden, wird daher im Stand der Technik vorgeschlagen, die heterogen katalysierte partielle Gasphasenoxidation von Acrolein zu Acrylsäure oder von Methacrolein zu Methacrylsäure, die Dehydrierung von Propan bzw. Isobutan zu Propen bzw. Isobuten, die Oxidation von Propen bzw. Isobuten zu Acrolein bzw. Methacrolein oder aber die Direktoxidation des Propans bzw. Isobutans zu Acrylsäure bzw. Methacrylsäure als Mehrzonenfahrweise (z. B. Zweizonenfahrweise) in einem Mehrzonenrohrbündelreaktor (z. B. in einem Zweizonenrohrbündelreaktor) ausführen. In diesem Fall werden im Reaktionsrohrumgebungsraum mehrere (z. B. zwei) voneinander im Wesentlichen räumlich getrennte flüssige Wärmeaustauschmittel (die normalerweise von derselben Sorte sind) geführt (diese können z. B. durch im Reaktionsrohrumgebungsraum eingezogene und für die Reaktionsrohre entsprechende Durchtrittsöffnungen aufweisende Trennrohrböden separiert sein).

Weiterhin besteht ein Nachteil herkömmlicher Rohrbündelreaktoren darin, dass Schwankungen in der Temperatur des Wärmeaustauschmittels sich sehr stark und unmittelbar auf die Temperatur im Reaktionsbereich der Reaktionsrohe auswirken, was leicht zu einem Durchgehen der Reaktoren führen kann. Auch weisen herkömmliche Rohrbündelreaktoren den Nachteil auf, dass die Hauptreaktion in den vorstehend beschriebenen *Hot*-*Spot*-Bereichen stattfindet, während im hinteren Reaktorteil (dieser macht etwa 2/3 der Gesamtlänge des Reaktors aus) die Temperatur für eine Reaktion meist zu niedrig ist. Mithin ist im hinteren Reaktorteil viel Katalysatormasse und Verweilzeit notwendig, um einen ausreichenden Umsatz sicherzustellen.

Schließlich führen die in den aus dem Stand der Technik bekannten Rohrbündelreaktoren eingesetzten Katalysator-Festbettschüttungen häufig zu einem starken Druckverlust, was eine Anpassung der Verdichterleistung erforderlich macht.

Der vorliegenden Erfindung lag die Aufgabe zugrunde, die sich aus dem Stand der Technik ergebenden Nachteile im Zusammenhang mit der Direktoxidation von gesättigten Kohlenwasserstoffen, mit der katalytischen Dehydrierung und nachfolgenden katalytischen Gasphasenoxidation von gesättigten Kohlenwasserstoffen sowie der katalytischen Gasphasenoxidation von ungesättigten Kohlenwasserstoffen oder ungesättigten Aldehyden ergebenden Nachteile zu überwinden.

Insbesondere lag der vorliegenden Erfindung die Aufgabe zugrunde, ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen anzugeben, bei dem Rohrbündelreaktoren mit einem im Vergleich zu den im Stand der Technik beschriebenen Reaktionsrohren deutlich größeren Durchmesser eingesetzt werden können, wobei dennoch eine Raum-Zeit-Ausbeute an (Meth)Acrylsäure erzielt werden kann, die vergleichbar mit der Raum-Zeit-Ausbeute der aus dem Stand der Technik bekannten Rohrbündelreaktoren ist. Auch sollte das Verfahren im Vergleich zu den aus dem Stand der Technik bekannten Verfahren deutlich sicherer betrieben werden können. Insbesondere der Bildung von *Hot Spots* oder die Gefahr des Durchgehens des Reaktors sollte deutlich geringer sein als bei den aus dem Stand der Technik bekannten Verfahren.

Auch lag der vorliegenden Erfindung die Aufgabe zugrunde, ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen anzugeben, welches im Vergleich zu den aus dem Stand der Technik bekannten Verfahren mit höherem Durchsatz betrieben werden kann.

Weiterhin lag der vorliegenden Erfindung die Aufgabe zugrunde, ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen anzugeben, bei dem ohne Einbußen hinsichtlich der Selektivität und/oder des Umsatzes auf eine Mehrzonenfahrweise gegebenenfalls verzichtet werden kann.

Der vorliegenden Erfindung lag darüber hinaus die Aufgabe zugrunde, ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen anzugeben, bei dem bei gleicher Verdichterleistung wie in den aus dem Stand der Technik bekannten Verfahren ein höherer Durchsatz erzielt werden kann. Weiterhin soll dieses Verfahren auch den Einsatz von mit Sauerstoff angereicherter Luft oder von reinem Sauerstoff in den Oxidationsschritten ermöglichen.

Auch lag der vorliegenden Erfindung die Aufgabe zugrunde, eine Vorrichtung zur Herstellung von ungesättigten Carbonsäuren anzugeben, mittels der sich die vorstehend beschriebenen Verfahrensvorteile erzielen lassen und welche im Vergleich zu den aus dem Stand der Technik bekannten Vorrichtungen mit geringen Investitionskosten bereitgestellt werden kann. Außerdem soll sich diese Vorrichtung deutlich einfacher mit dem Katalysator befüllen lassen.

Einen Beitrag zur Lösung der eingangs genannten Aufgaben leistet ein Verfahren zur Herstellung von ungesättigten Aldehyden oder ungesättigten Carbonsäuren durch heterogene, katalytische Gasphasenoxidation von gesättigten oder ungesättigten Kohlenwasserstoffen, beinhaltend die Verfahrensschritte:
i) Bereitstellung eines Gasgemisches mindestens beinhaltend einen gesättigten Kohlenwasserstoff und katalytische Gasphasendehydrierung des mindestens einen gesättigten Kohlenwasserstoffs unter Erhalt eines einen ungesättigten Kohlenwasserstoff enthaltenen Gasgemisches in einem ein Dehydrier-Katalysatormaterial aufweisenden Dehydrier-Reaktor; oder
ii) Bereitstellung eines Gasgemisches mindestens beinhaltend Sauerstoff und mindestens einen ungesättigten Kohlenwasserstoff;
iii) katalytische Gasphasenoxidation des in Verfahrensschritt i) erhaltenen oder des in Verfahrensschritt ii) bereitgestellten ungesättigten Kohlenwasserstoffs unter Erhalt eines einen ungesättigten Aldehyd enthaltenen Gasgemisches in einem ersten, ein erstes Oxidations-Katalysatormaterial aufweisenden Oxidations-Reaktor;
iv) gegebenenfalls katalytische Gasphasenoxidation des im Verfahrensschritt iii) erhaltenen, ungesättigten Aldehyds unter Erhalt eines eine ungesättigte Carbonsäure enthaltenen Gasgemisches in einem zweiten, ein zweites Oxidations-Katalysatormaterial aufweisenden Oxidations-Reaktor;
wobei mindestens einer der Reaktoren ausgewählt aus dem Dehydrier-Reaktor, dem ersten Oxidations-Reaktor und dem zweiten Oxidations-Reaktor mindestens einen offenzelligen, metallischen oder keramischen Schaumkörper beinhaltet und der Schaumkörper durch eine Zellweite in einem Bereich von 39,4 bis 1574,8 Poren pro Meter (1 bis 40 ppi) gekennzeichnet ist. Der Schaumkörper kann entweder zur Mehrheit aus keramischen oder metallischen Material oder auch einer Mischung aus keramischen und metallischen Material bestehen, wobei entweder keramisches oder metallisches Material bevorzugt ist. Gleiches gilt auch für die erfindungsgemäßen Verfahren.

Neben offenzelligen Schäumen sind dem Fachmann geschlossenzellige Schäume bekannt. Bei geschlossenzelligen Schaum sind die Wände zwischen der überwiegenden Zahl, oftmals mehr als 80 % oder im wesentlichen jede, der einzelnen Zellen im Schaumkörpers komplett geschlossen. Bei offenzelligem Schaum sind die Zellwände der meisten Zellen, oftmals mehr als 50 % oder im wesentlichen jeder, im Schaumkörper nicht geschlossen. Diese Schaumstoffe können daher Flüssigkeiten oder Gase besser aufnehmen oder leiten als geschlossenenzellige Schäume, weil die meisten, oftmals mehr als 50 % der Zellen im Schaumkörper miteinander verbunden sind.

Völlig überraschend, dafür aber nicht minder vorteilhaft, wurde festgestellt, dass durch den Einsatz von offenzelligen, metallischen oder keramischen Schaumkörpern in den Dehydrier und/oder Oxidations-Reaktoren größere Durchsätze bei gleichen Reaktordimensionen erzielt werden können und dass sich zudem die Lebensdauer der eingesetzten Katalysatoren deutlich erhöhen lässt. Die durch den Einsatz von offenzelligen Schaumkörpern in den Dehydrier- und/oder Oxidations-Reaktoren resultierenden, niedrigeren Heisspunkttemperaturen ermöglichen zudem größere Rohrduchmesser und mithin geringe Investivkosten. Weiterhin kann durch den Einsatz von offenzelligen Schaumkörpern in den Oxidations-Reaktoren auf den Einsatz von Katalysatorschüttungen unterschiedlicher Aktivität in axialer Richtung, auf den Einsatz von verschiedenen Kühlkreisen oder auf den Einsatz unterschiedlicher Katalysatormaterialien verzichtet werden, um einen stabilen Betrieb im Reaktor zu gewährleisten. Weiterhin ist der Einsatz von offenzelligen Schaumkörpern in den Dehydrier- und/oder Oxidations-Reaktoren mit einem im Vergleich zu herkömmlichen Festbettschüttungen geringeren Druckverlust verbunden, so dass sich im Vergleich zu herkömmlichen Rohrbündelreaktoren höhere Durchsätze bei gleicher Verdichterleistung erzielen lassen.

Gemäß einer besonderen Ausgestaltung des erfindungsgemäßen Verfahrens kann der gesättigte Kohlenwasserstoff auch in einer Direktoxidation zur ungesättigten Carbonsäure umgesetzt werden (so dass die Verfahrensschritte i), iii) und iv) zeitgleich in ein und demselben Reaktor ablaufen). Der Begriff "*Direktoxidation*" soll dabei zum Ausdruck bringen, dass es beim Durchgang des den gesättigten Kohlenwasserstoff enthaltenden Reaktionsgasgemisches durch das Katalysatorbett jeweils unmittelbar zur Ausbildung der ungesättigten Carbonsäure kommt. Das Katalysatorbett ist demnach nicht so beschaffen, dass beim Durchströmen des den gesättigten Kohlenwasserstoff enthaltenden Reaktionsgasgemisches durch das Katalysatorbett in dessen in Strömungsrichtung ersten Längsabschnitten zunächst hauptsächlich isolierbare Zwischenprodukte, wie zum Beispiel ungesättigte Kohlenwasserstoffe oder ungesättigte Aldehyde, gebildet werden, aus denen erst in den sich in Strömungsrichtung anschließenden weiteren Längsabschnitten des Katalysatorbetts die ungesättigte Carbonsäure entstehen würde. Vielmehr sind wenigstens einzelne im Katalysatorbett mitverwendeten Katalysatormaterialien befähigt, jeweils alle der verschiedenen auf dem Reaktionsweg vom gesättigten. Kohlenwasserstoff zur ungesättigten Carbonsäure zurückzulegenden Reaktionsschritte zu katalysieren, so dass am jeweiligen der so befähigten Katalysatorenma-terialien beispielsweise unmittelbar eine Acrylsäurebildung aus Propan oder eine Methacrylsäurebildung aus Isobutan erfolgten kann bzw. erfolgt. In diesem Falle wird daher nur ein einziger Oxidations-Reaktor eingesetzt und das Dehydrier-Katalysatormaterial, das erste Oxidations-Katalysatormaterial und das zweite Oxidations-Katalysatormaterial sind entweder identisch oder liegen zumindest gemeinsam, gegebenenfalls als Gemisch, in diesem einen Reaktor vor oder es wird ein Katalysatormaterial verwendet, welches in der Lage ist, alle der vorstehend genannten Reaktionsschritte zu katalysieren oder über einen anderen Reaktionsweg zum Zielprodukt (ungesättigte Carbonsäure) umzusetzen.

Gemäß einer ersten besonderen Ausführungsform des erfindungsgemäßen Verfahrens umfasst das Verfahren die Verfahrensschritte ii), iii) und iv). Bei dieser Ausführungsform handelt es sich bei dem ungesättigten Kohlenwasserstoff um Propen, bei dem ungesättigten Aldehyd um Acrolein und bei der ungesättigten Carbonsäure um Acrylsäure. Im Zusammenhang mit dieser Ausführungsform ist es insbesondere bevorzugt, dass der Dehydrier-Reaktor, der erste Oxidations-Reaktor, der zweite Oxidations-Reaktor, der Dehydrier-Reaktor und der erste Oxidations-Reaktor, der Dehydrier-Reaktor und der zweite Oxidations-Reaktor, der erste Oxidations-Reaktor und der zweite Oxidations-Reaktor oder der Dehydrier-Reaktor, der erste Oxidations-Reaktor und der zweite Oxidationsreaktor mindestens einen offenzelligen, metallischen oder keramischen Schaumkörper beinhaltet.

Gemäß einer zweiten besonderen Ausführungsform des erfindungsgemäßen Verfahrens umfasst das Verfahren ebenfalls die Verfahrensschritte ii), iii) und iv). Bei dieser Ausführungsform handelt es sich bei dem ungesättigten Kohlenwasserstoff um Isobuten, bei dem ungesättigten Aldehyd um Methacrolein und bei der ungesättigten Carbonsäure um Methacrylsäure. Im Zusammenhang mir dieser Ausführungsform ist es insbesondere bevorzugt, dass der Dehydrier-Reaktor, der erste Oxidations-Reaktor, der zweite Oxidations-Reaktor, der Dehydrier-Reaktor und der erste Oxidations-Reaktor, der Dehydrier-Reaktor und der zweite Oxidations-Reaktor, der erste Oxidations-Reaktor und der zweite Oxidations-Reaktor oder der Dehydrier-Reaktor, der erste Oxidations-Reaktor und der zweite Oxidationsreaktor mindestens einen offenzelligen, metallischen oder keramischen Schaumkörper beinhaltet.

Gemäß einer dritten besonderen Ausführungsform des erfindungsgemäßen Verfahrens umfasst das Verfahren die Verfahrensschritte i), ii), iii) und iv). Bei dieser Ausführungsform handelt es sich bei dem gesättigten Kohlenwasserstoff um Propan, bei dem ungesättigten Kohlenwasserstoff um Propen, bei dem ungesättigten Aldehyd um Acrolein und bei der ungesättigten Carbonsäure um Acrylsäure. Im Zusammenhang mir dieser Ausführungsform ist es insbesondere bevorzugt, dass der Dehydrier-Reaktor, der erste Oxidations-Reaktor, der zweite Oxidations-Reaktor, der Dehydrier-Reaktor und der erste Oxidations-Reaktor, der Dehydrier-Reaktor und der zweite Oxidations-Reaktor, der erste Oxidations-Reaktor und der zweite Oxidations-Reaktor oder der Dehydrier-Reaktor, der erste Oxidations-Reaktor und der zweite Oxidationsreaktor mindestens einen offenzelligen, metallischen oder keramischen Schaumkörper beinhaltet. Zu beachten ist, dass gemäß einer besonderen Ausgestaltung dieser dritten besonderen Ausführungsform des erfindungsgemäßen Verfahrens das Propen bzw. das Isobutan auch durch eine Direktoxidation zu Acrylsäure bzw. zu Methacrylsäure umgesetzt werden kann.

Gemäß einer vierten besonderen Ausführungsform des erfindungsgemäßen Verfahrens umfasst das Verfahren die Verfahrensschritte i), ii), iii) und iv). Bei dieser Ausführungsform handelt es sich bei dem gesättigten Kohlenwasserstoff um Isobutan, bei dem ungesättigten Kohlenwasserstoff um Isobuten, bei dem ungesättigten Aldehyd um Methacrolein und bei der ungesättigten Carbonsäure um Methacrylsäure. Im Zusammenhang mir dieser Ausführungsform ist es insbesondere bevorzugt, dass der Dehydrier-Reaktor, der erste Oxidations-Reaktor, der zweite Oxidations-Reaktor, der Dehydrier-Reaktor und der erste Oxidations-Reaktor, der Dehydrier-Reaktor und der zweite Oxidations-Reaktor, der erste Oxidations-Reaktor und der zweite Oxidations-Reaktor oder der Dehydrier-Reaktor, der erste Oxidations-Reaktor und der zweite Oxidationsreaktor mindestens einen offenzelligen, metallischen oder keramischen Schaumkörper beinhaltet. Auch hier ist zu beachten, dass gemäß einer besonderen Ausgestaltung dieser vierten besonderen Ausführungsform des erfindungsgemäßen Verfahrens das Isobuten auch durch eine Direktoxidation zu Methacrylsäure umgesetzt werden kann.

Im Verfahrensschritt i) des erfindungsgemäßen Verfahrens wird gegebenenfalls zunächst ein Gasgemisch mindestens beinhaltend einen gesättigten Kohlenwasserstoff bereitgestellt und der in diesem Gasgemisch enthaltene Kohlenwasserstoff durch katalytische Gasphasenoxidation unter Erhalt eines einen ungesättigten Kohlenwasserstoffs enthaltenen Gasgemisches in einem ein Dehydrier-Katalysatormaterial aufweisenden Dehydrier-Reaktor dehydriert und mithin oxidiert. Bei diesem gesättigten Kohlenwasserstoff handelt es sich vorzugsweise um Propan oder um Isobutan (siehe die vorstehend beschriebene, dritte bzw. vierte besondere Ausführungsform des erfindungsgemäßen Verfahrens).

Verfahren zur Herstellung von Acrolein oder Acrylsäure aus Propan, bei denen man in einer ersten Reaktionszone Propan heterogen katalysiert partiell zu Propen dehydriert und nachfolgend das gebildete Propen zu Acrolein, oder zu Acrylsäure, oder zu deren Gemisch oxidiert, sind unter anderem aus DE-A-33 13 573, EP-A-0 117146, US-A-3,161,670, DE-A-10 2004 032 129, EP-A-0 731 077, DE-A-10 2005 049 699, DE-A-10 2005 052 923, WO-A-01/96271, WO-A-03/011804, WO-A-03/076370, WO-A-01/96270, DE-A-10 2005 009 891, DE-A-10 2005 013 039, DE-A-10 2005 022 798, DE-A-10 2005 009 885, DE-A-10 2005 010 111 und DE-A-102 455 85 bekannt. Ebenfalls sind Verfahren bekannt, bei denen man in einer ersten Reaktionszone Isobutan heterogen partiell zu Isobuten dehydriert und nachfolgend das gebildete Isobuten zu Methacrolein, oder zu Methacrylsäure, oder zu deren Gemisch oxidiert (siehe zum Beispiel DE-A-33 13 573).

Grundsätzlich kann dabei die Dehydrierung des gesättigten Kohlenwasserstoffs in Gegenwart von Sauerstoff als exotherme Oxidehydrierung (der dem Dehydrieren entrissene Wasserstoff reagiert mit dem Sauerstoff unmittelbar zu Wasser) oder aber als endotherme Dehydrierung in Abwesenheit von Sauerstoff (beim Dehydrieren entsteht molekularer Wasserstoff) durchgeführt werden.

Als Dehydrier-Katalysatormaterialien kommen grundsätzlich alle im Stand der Technik bekannten Dehydrier-Katalysatorenmaterialien in Betracht. Sie lassen sich grob in zwei Gruppen unterteilen. Solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrier-Katalysatormaterialien eingesetzt werden, die in der WO-A-01/96270, der EP-A-0 731 077, der DE-A-102 11 275, der DE-A 101 31 297, der WO-A-99/46039, der US 4,788,371, der EP-A-0 705 136, der WO-A-99/29420, der US 4,220,091, der US 5,430,220, der US 5,877,369 der EP-A-0 117 146, der DE-A-199 37 196, der DE-A-199 37 105, der US-A-3,670,044, der US 6,566,573, der US 4,788,371 der WO-A-94/29021 oder der DE-A-199 37 107 beschrieben werden. Erfindungsgemäß besonders bevorzugt sind Dehydrier-Katalysatorenmaterialien auf Platin- oder auf Platin-Zinn-Basis, die auf geeigneten Trägermaterialien, wie beispielsweise den offenzelligen, metallischen oder keramischen Schaumkörper, aufgebracht werden können.

Zur heterogen katalysierten Dehydrierung von Propan oder Isobuten im Verfahrensschritt i) kommen darüber hinaus alle im Stand der Technik bekannten Reaktortypen und Verfahrensvarianten in Betracht. Eine vergleichsweise ausführliche Beschreibung von für geeignete Dehydrierverfahren enthält auch Catalytica^{®} Studies Division, "Oxidative Dehydrogenation and Alternative Dehydrogenation Processes", Study Number 4192 OD, 1993, 430 Ferguson Drive, Mountain View, California, 94043-5272 U.S.A..

Im Verfahrensschritt ii) des erfindungsgemäßen Verfahrens wird ein Gasgemisch mindestens beinhaltend Sauerstoff und mindestens ein ungesättigten Kohlenwasserstoff bereitgestellt, wobei es sich bei diesem ungesättigten Kohlenwasserstoff vorzugsweise um Propen oder Isobuten handelt. Neben dem ungesättigten Kohlenwasserstoff und dem Sauerstoff kann dieses Gasgemisch weiterhin Inertgase, wie etwa Stickstoff, sowie Wasserdampf enthalten. Die genaue Zusammensetzung derartiger, bei der katalytischen Gasphasenoxidation von Propen oder Isobuten eingesetzter Ausgangs-Gasgemische ist dem Fachmann aus dem Stand der Technik hinreichend bekannt. Im Falle eines Einsatzes von Propen als ungesättigter Kohlenwasserstoff wird beispielsweise auf die WO-A-03/051809 verwiesen, in der die genaue Zusammensetzung eines Propen beinhaltenden Gasgemisches, welches in einer zweistufigen Oxidationsreaktion zugeführt werden kann, beschrieben ist.

Im Verfahrensschritt iii) des erfindungsgemäßen Verfahrens wird der in Verfahrensschritt i) erhaltene oder der in Verfahrensschritt ii) bereitgestellte ungesättigten Kohlenwasserstoff in der Gasphase unter Erhalt eines einen ungesättigten Aldehyd enthaltenen Gasgemisches in einem ersten, ein erstes Oxidations-Katalysatormaterial aufweisenden Oxidations-Reaktor oxidiert, während gegebenenfalls in Verfahrensschritt iv) des erfindungsgemäßen Verfahrens der im Verfahrensschritt iii) erhaltene, ungesättigte Aldehyd in der Gasphase unter Erhalt eines eine ungesättigte Carbonsäure enthaltenen Gasgemisches in einem zweiten, ein zweites Oxidations-Katalysatormaterial aufweisenden Oxidations-Reaktor oxidiert wird. Die hierzu in den einzelnen Reaktionsstufen vorteilhafterweise eingesetzten Katalysatormaterialien und die vorteilhaften Druck- und Temperaturbedingungen sind im Falle der zweistufigen Umsetzung von Propen zu Acrylsäure ebenfalls der WO-A-03/051809 zu entnehmen. Weitere geeignete Katalysatormaterialien insbesondere für die Umsetzung von Propen zu Acrolein und von Acrolein zu Acrylsäure und auch die bevorzugten Druck- und Temperaturbedingungen für diese Umsetzungen können darüber hinaus auch der WO-A-2006/002708 entnommen werden.

Besonders bevorzugt ist es, dass als Katalysatormaterial für die katalytische Oxidationsreaktion von Propen zu Acrolein Mo-basierte Katalysatormaterialien, noch mehr bevorzugt Mo-Bi-basierte Katalysatormaterialien verwendet werden, wobei Mo-Fe-Bi-basierte Katalysatormaterialien am meisten bevorzugt sind. Als Nebenkomponenten derartiger Katalysatorsysteme kommen weiterhin insbesondere Co/Ni, Sb, Alkalimetalle, Erdalkalimetalle, Zn, Sn, As, Pb, Pd und verschiedene andere, meist Übergangsmetalle in Betracht. Weiterhin können diese Katalysatormassen beispielsweise zur Regulierung der Katalysatoraktivität auch mit Inert-Zusätzen dotiert sein, wobei hier beispielsweise Al₂O₃, SiO₂, ZrO₂, TiO₂ und Mischungen bzw. Verbindungen davon zur Anwendung kommen können. Für die katalytische Oxidation des Isobutans zum Methacrolein werden vorzugsweise ebenfalls die vorstehend beschriebenen Katalysatormaterialien eingesetzt.

In der Stufe der katalytischen Oxidationsreaktion vom Acrolein zu Acrylsäure werden besonders bevorzugt Mo-basierte Katalysatormaterialien, noch mehr bevorzugt Mo-V-basierte Katalysatormaterialien verwendet, wobei hier Mo-Cu-V-basierte oder Mo-W-V-basierte Katalysatormaterialien am meisten bevorzugt sind. Weiterhin ist es bevorzugt, dass die Katalysatormaterialien vom Mischoxid-Typ sind. Als Nebenkomponenten dieser Katalysatorsysteme kommen insbesondere weitere Übergangsmetalle in Betracht. Weiterhin können auch diese Katalysatormaterialien beispielsweise zur Regulierung der Katalysatoraktivität mit Inert-Zusätzen dotiert sein, wobei hier ebenfalls Al₂O₃, SiO₂, ZrO₂, TiO₂ und Mischungen bzw. Verbindungen davon zur Anwendung kommen können. Für die katalytische Oxidation des Methacroleins zur Methacrylsäure werden vorzugsweise neben den vorstehend beschriebenen Katalysatormaterialien auch auf Mo-V-P-basierende Katalysatormaterialien eingesetzt.

Sofern, wie eingangs beschrieben, das Propan bzw. das Isobutan durch eine Direktoxidation zu Acrylsäure bzw. zu Methacrylsäure umgesetzt wird, so können als Katalysatormaterialien solche der Zusammensetzung Mo-V-Te-(Nebenkomponenten)-O eingesetzt werden, wobei als Nebenkomponenten insbesondere Nb eingesetzt werden kann. Auch diese Katalysatormaterialien können beispielsweise zur Regulierung der Katalysatoraktivität mit Inert-Zusätzen dotiert sein, wobei hier beispielsweise Al₂O₃, SiO₂, ZrO₂, TiO₂ und Mischungen bzw. Verbindungen davon zur Anwendung kommen können.

Die Belastung an ungesättigtem Kohlenwasserstoff, vorzugsweise die Propen- bzw. Isobuten-Belastung, in dem erfindungsgemäßen Verfahren (also die Menge an ungesättigtem Kohlenwasserstoff, vorzugsweise an Propen bzw. Isobuten in Litern, die pro Liter Reaktorvolumen und pro Stunde in den ersten Oxidations-Reaktor eingebracht wird) liegt vorzugsweise bei mindestens 150/h, besonders bevorzugt bei mindestens 200/h, darüber hinaus bevorzugt mindestens 250/h und am meisten bevorzugt mindestens 300/h.

Gemäß dem erfindungsgemäßen Verfahren beinhaltet nun mindestens einer der Reaktoren ausgewählt aus dem Dehydrier-Reaktor (in dem die Umsetzung des gesättigten Kohlenwasserstoffs, vorzugsweise des Propans oder des Isobutans, zum ungesättigten Kohlenwasserstoff, vorzugsweise zu Propen oder Isobuten, erfolgt), dem ersten Oxidations-Reaktor (in dem die Umsetzung des ungesättigten Kohlenwasserstoffs, vorzugsweise des Propens oder des Isobutens, zum ungesättigten Aldehyd, vorzugsweise zu Acrolein bzw. Methacrolein erfolgt), dem zweiten Oxidations-Reaktor (in dem die Umsetzung des ungesättigten Aldehyds, vorzugsweise des Acroleins oder Methacroleins, zur ungesättigten Carbonsäure, vorzugsweise zu Acrylsäure bzw. Methacrylsäure erfolgt) oder dem Reaktor zur Direktoxidation (in dem die Umsetzung des gesättigten Kohlenwasserstoffs, vorzugsweise des Propans oder des Isobutans, zur ungesättigten Carbonsäure, vorzugsweise zu Acrylsäure bzw. Methacrylsäure erfolgt) mindestens einen offenzelligen metallischen oder keramischen Schaumkörper.

Erfindungsgemäß sind dabei folgende Varianten des erfindungsgemäßen Verfahrens denkbar:
- nur der Dehydrier-Reaktor beinhaltet einen offenzelligen, metallischen oder keramischen Schaumkörper;
- nur der erste Oxidations-Reaktor beinhaltet einen offenzellige, metallischen oder keramischen Schaumkörper;
- nur der zweite Oxidations-Reaktor beinhaltet einen offenzellige, metallischen oder keramischen Schaumkörper;
- der Dehydrier-Reaktor und der erste Oxidations-Reaktor beinhalten einen offenzelligen, metallischen oder keramischen Schaumkörper;
- der Dehydrier-Reaktor und der zweite Oxidations-Reaktor beinhalten einen offenzelligen, metallischen oder keramischen Schaumkörper;
- der erste Oxidations-Reaktor und der zwei Oxidations-Reaktor beinhalten einen offenzelligen, metallischen oder keramischen Schaumkörper;
- der Dehydrier-Reaktor, der erste Oxidations-Reaktor und der zweite Oxidations-Reaktor beinhalten einen offenzelligen, metallischen oder keramischen Schaumkörper;
- der Reaktor zur Direktoxidation beinhaltet einen offenzelligen, metallischen oder keramischen Schaumkörper.

Als offenzellige, metallische oder keramische Schaumkörper kommen dabei alle dem Fachmann bekannten offenzelligen, metallischen oder keramischen Schaumkörper in Betracht, welche unter den Druck- und Temperaturbedingungen im Dehydrier-Reaktor und/oder im ersten Oxidations-Reaktor und oder dem zweiten Oxidations-Reaktor eingesetzt werden können.

Als offenzellige metallische Schäume kommen beispielsweise Aluminiumschäume oder Schäume aus Stahl, Edelstahl oder Kupfer in Betracht. Die Herstellung solcher Schwämme aus Metallen ist beispielsweise in der US 3,087,807 oder in der DE-A-40 18 360 beschrieben. Auch die offenzelligen keramischen Schäume bestehen vorzugsweise aus einer dreidimensional vernetzten Keramikstruktur aus vielen miteinander verbundenen Keramikstegen und zwischen den Stegen liegenden offenen Zellen. Die Art der Keramik, aus der die Stege aufgebaut sind, lässt sich variieren und bestimmt wesentliche Eigenschaften des Schaumes. Bevorzugt Offenzellige Schaumkeramiken werden hauptsächlich durch Abformungsverfahren hergestellt, bei dem eine bevorzugt offenzellige Gerüststruktur, z. B. aus Polymerschaum oder Kohlenstoffschaum, mit Keramik beschichtet wird und das ursprüngliche Substrat entweder während oder nach der Keramikausbildung entfernt wird. Ein solches Verfahren ist beispielsweise in der US 3,090,094 beschrieben. Vorzugsweise basieren die keramischen Schaumkörper auf Mischoxiden oder Carbiden. Als Material für erfindungsgemäß verwendbare keramische Schaumkörper finden beispielsweise Materialien wie Cordient, Steatit, Duranit^{®}, Siliziumcarbid oder oxidisch gebundenes Siliziumcarbid oder Schaumkörper aus Siliziumdioxid, Aluminiumoxiden, Aluminosilikaten oder Aluminaten Verwendung. Als Beispiel für ein Verfahren zur Herstellung eines bevorzugt offenzelligen, auf Siliziumcarbit basierenden, keramischen Schaumes sei das in der WO-A-02/020426 beschriebene Verfahren genannt. Es ist erfindungsgemäß weiterhin bevorzugt, dass die metallischen oder keramischen Schaumkörper eine Zellweite in einem Bereich von 196,9 bis 1574,8 Poren pro Meter (5 bis 40 ppi ("*pores per inch*")) und besonders bevorzugt in einem Bereich von 393,7 bis 1574,8 Poren pro Meter (10 bis 40 ppi) aufweisen.

Gemäß einer Ausführungsform des erfindungsgemäßen Verfahrens sind die offenzelligen, metallischen oder keramischen Schaumkörper nicht mit dem Dehydrier-Katalysatormaterial, dem ersten Oxidations-Katalysatormaterial oder dem zweiten Oxidations-Katalysatormaterial belegt, sondern sind zusätzlich zu entsprechenden Festbettschüttungen in den jeweiligen Reaktoren enthalten.

Gemäß einer bevorzugten Ausführungsform des erfindungsgemäßen Verfahrens ist jedoch mindestens ein Teil der Oberfläche des offenzelligen, metallischen oder keramischen Schaumkörpers mit dem Dehydrier-Katalysatormaterial, dem ersten Oxidations-Katalysatormaterial, dem zweiten Oxidations-Katalysatormaterial (je nachdem, ob sich der Schaumkörper im Dehydrier-Reaktor, im ersten Oxidations-Reaktor oder im zweiten Oxidations-Reaktor befindet) oder dem für die Durchführung einer Direktoxidation geeigneten Katalysatormaterial belegt. Der offenzellige, metallische oder keramische Schaumkörper dient in diesem Fall als Trägermaterial für das im jeweiligen Reaktor (Dehydrier-Reaktor, erster Oxidations-Reaktor bzw. zweiter Oxidations-Reaktor) eingesetzte Katalysatormaterial. Weiterhin ist jedoch auch eine Ausführungsform denkbar, bei welcher der offenzellige Schaumkörper selbst aus einem Material mit entsprechender katalytischer Aktivität besteht, in dem er beispielsweise aus diesem geformt wurde.

Das Belegen des offenzelligen, metallischen oder keramischen Schaumkörpers mit dem Dehydrier-Katalysator, dem ersten Oxidations-Katalysator oder dem zweiten Oxidations-Katalysator (also beispielsweise mit dem Mo-basierten Katalysatormaterial, besonders bevorzugt dem Mo-Bi-basierten Katalysatormaterial und am meisten bevorzugt mit dem Mo-Fe-Bi-basierten Katalysatormaterial der ersten Oxidationsstufe bzw. mit dem Mo-basierten Katalysatormaterial, besonders bevorzugt dem Mo-V-basierten Katalysatormaterial und am meisten bevorzugt mit dem Mo-Cu-V-basierten, dem Mo-W-V-basierten oder dem Mo-V-P-basierten Katalysatormaterial der zweiten Oxidationsstufe) erfolgt vorzugsweise durch das Aufbringen einer Lösung oder einer Suspension des Katalysatormaterials auf die Oberflache seiner inneren Hohlräume, gefolgt von einer Trocknung mit anschließender Kalzinierung bei höheren Temperaturen zur Verfestigung und endgültigen Oberflächengestaltung des aufgebrachten Katalysatormaterials.

Gemäß einer bevorzugten Ausführungsform des erfindungsgemäßen Verfahrens zur Herstellung von ungesättigten Aldehyden oder ungesättigten Carbonsäuren ist der mit dem Katalysator zumindest teilweise belegte offenzellige, metallische oder keramische Schaumkörper demnach erhältlich durch ein Verfahren beinhaltend die Verfahrensschritte:
A1) Herstellung einer Lösung oder einer Suspension aus dem vorzugsweise pulverförmigen Katalysatormaterial, einem Lösungsmittel oder einem Lösungsmittelgemisch sowie gegebenenfalls weiteren Additiven;
A2) In Kontakt bringen mindestens eines Teils der Oberfläche des offenzelligen, metallischen oder keramischen Schaumkörpers mit der Lösung bzw. der Suspension;
A3) gegebenenfalls Austragen eines Überschusses an Lösung bzw. Suspension;
A4) gegebenenfalls Trocknen des offenzelligen, metallischen oder keramischen Schaumkörpers;
A5) Kalzinieren des Katalysatormaterials.

Im Verfahrensschritt A1) wird zunächst eine Lösung bzw. Suspension des vorzugsweise pulverförmigen Katalysatormaterials in einem geeigneten Lösungsmittel oder Lösungsmittelgemisch, beispielsweise Wasser, ein- oder mehrwertige, Alkohole oder Mischungen daraus, hergestellt, gegebenenfalls unter Zusatz von Additiven, wie anorganischen oder organischen Bindern, Tensiden, katalytischen Aktivkomponenten, Porenbildnern, Rheologiehilfsmitteln, Inert-Materialien zur Regulierung der Katalysator-Aktivität, Haftvermittlern, Entschäumern, Dispergierhilfsmitteln und anderen Zusatzstoffen. Im Verfahrensschritt A2) wird dann mindestens ein Teil der Oberfläche des offenzelligen, metallischen oder keramischen Schaumkörpers mit der Lösung bzw. Suspension in Kontakt gebracht. Dieses kann beispielsweise dadurch geschehen, dass der Schaumkörper durch einen Tauch-, Saug-, Streich- oder Pumpprozess mit der Lösung bzw. Suspension befüllt wird. Im Stand der Technik sind Verfahren beschrieben, bei denen nur die exakt berechnete und in dem Schaumkörper zu verbleibende Menge an Lösung bzw. Suspension in den Schaumkörper eingebracht wird und diese Menge möglichst gleichmäßig auf die äußeren und inneren Oberflächen des Schaumkörpers verteilt wird. Andere Verfahren geben einen Überschuss in den Schaumkörper (z. B. Flutung des Schaumkörpers) ein und führen einen anschließenden Entleerungsvorgang durch, mit dem überschüssige Lösung bzw. Suspension gemäß dem Verfahrensschritt A3) ausgetragen wird. Oft wird zur Entleerung ein Ausblasen mittels eines Luftstromes durchgeführt, wobei das Entleeren auch mittels Rotation des Schaumkörpers oder durch Absaugen möglich ist. In Verfahrensschritt A4) wird dann der offenzellige, metallische oder keramische Schaumkörper getrocknet, wobei diese Trocknung vorzugsweise bei Temperaturen in einem Bereich von 50 bis 200°C, besonders bevorzugt in einem Bereich von 100 bis 150°C erfolgt. Denkbar ist dabei, die Verfahrenschritte A1) bis A3), insbesondere auch die Verfahrensschritte A1) bis A4) mehrfach hintereinander durchzuführen, um auf diese Weise beispielsweise die Dicke der aufgebrachten Schicht aus dem Katalysatormaterial variieren zu können.

Im Verfahrensschritt A5) wird das auf der Oberfläche des Schaumkörpers befindliche Katalysatormaterial dann kalziniert, wobei dieses Kalzinieren vorzugsweise durch Erhitzen des mit dem Katalysatormaterial belegten Schaumkörpers auf Temperaturen in einem Bereich von 250 bis 750°C, besonders bevorzugt in einem Bereich von 300 bis 600°C und am meisten bevorzugt in einem Bereich von 350 bis 500°C erfolgt. Vorzugsweise erfolgt das Kalzinieren durch Hindurchleiten eines Gasstroms, beispielsweise von N₂ und/oder Luft.

Gemäß einer weiteren, besonderen Ausführungsform des erfindungsgemäßen Verfahrens handelt es sich bei mindestens einen der Reaktoren ausgewählt aus dem Dehydrier-Reaktor, dem ersten Oxidations-Reaktor, dem zweiten Oxidations-Reaktor oder demjenigen Reaktor, in dem eine Direktoxidation durchgeführt wird, um einen Rohrbündelreaktor umfassend mindestens zwei Reaktionsrohre, wobei das Dehydrier-Katalysatormaterial, das erste Oxidations-Katalysatormaterial, das zweite Oxidations-Katalysatormaterial oder das die Direktoxidation katalysierende Material und der offenzellige Schaumkörper bzw. der mit dem Dehydrier-Katalysatormaterial, dem ersten Oxidations-Katalysatormaterial, dem zweiten Oxidations-Katalysatormaterial bzw. dem die Direktoxidation katalysierenden Material belegte offenzellige Schaumkörper bzw. der aus dem Dehydrier-Katalysatormaterial, dem ersten Oxidations-Katalysatormaterial, dem zweiten Oxidations-Katalysatormaterial bzw. dem die Direktoxidation katalysierenden Material geformte, offenzellige Schaumkörper im Inneren der Reaktionsrohre lokalisiert ist/sind. Solche Rohrbündelreaktoren umfassen ein in der Reaktorhülle angeordnetes, aus einer Vielzahl paralleler Reaktorrohre aufgebautes Rohrbündel. Die Reaktorrohre sind mit ihren offenen Enden in einem oberen und einem unteren Rohrboden abdichtend befestigt und münden in jeweils eine obere und eine untere Reaktorhaube. Über die Reaktorhauben wird das Edukt beziehungsweise das die Reaktorrohre verlassende Produktgemisch zu- beziehungsweise abgeführt. Gemäß einer besonderen Ausführungsform des erfindungsgemäßen Verfahrens können mindestens zwei, drei, vier, fünf oder noch mehr solcher Rohrbündelreaktoren parallel zueinander betrieben werden.

In dem Raum zwischen dem oberen und dem unteren Rohrboden, der durch Umlenkbleche unterteilt sein kann, zirkuliert ein Wärmeträger, welcher im Gleich-, Gegenstrom oder Querstrom geführt werden kann. In der Reaktorhülle sind entsprechende Zu- und Abführungen für das Wärmeträgermedium angeordnet, durch die das Wärmeträgermedium mittels Pumpen im Kreislauf geführt wird, und wobei mittels außenliegender Wärmetauscher eine Solltemperatur eingestellt wird. Die Reaktorhülle kann in zwei oder mehr unabhängige Temperierzonen unterteilt sein, die jeweils mit voneinander unabhängig zirkulierenden Wärmeträgermedien betrieben werden. Die Trennung der einzelnen Zonen voneinander erfolgt durch Rohrböden, welche horizontal im Rohrbündelreaktor angeordnet sind und durch welche die Reaktorrohre geführt werden.

Die einzelnen Rohre des Rohrbündelreaktors weisen dabei vorzugsweise einen Durchmesser von mehr als 3 cm, besonders bevorzugt mehr als 5 cm und noch mehr bevorzugt mehr als 10 cm auf.

Weiterhin ist es neben der vorstehend beschriebenen, besonderen Ausführungsform des erfindungsgemäßen Verfahrens auch denkbar, Wandreaktoren oder Plattenreaktoren einzusetzen. In Wandreaktoren wird ein Reaktionsgemisch zwischen jeweils zwei katalytisch beschichteten parallel angeordneten plattenförmigen Wandelementen hindurchgeleitet, wobei das Katalysatormaterial (Dehydrier-Katalysatormaterial, erstes Oxidations-Katalysatormaterial, zweites Oxidations-Katalysatormaterial bzw. die Direktoxidation katalysierendes Material) und der Schaumkörper, der mit dem Katalysatormaterial belegte Schaumkörper oder der aus diesem Katalysatormaterial geformte in dem Reaktionsraum zwischen zwei benachbarten Platten lokalisiert ist. Üblicherweise besteht ein solcher Reaktor aus einer Reihe von Wandelementen. Aufgrund des geringen Wandelementabstandes wird ein hohes Wand- zu Volumenverhältnis erzielt, welches eine hohe Wärmeabfuhrrate und eine Fahrweise mit unter normalen Bedingungen explosionsfähigen Reaktionsgemischen erlaubt. Die hohe Wärmeabfuhrrate ermöglicht eine sehr gute Temperaturkontrolle unter Vermeidung von "*hot spots*" bei stark exothermen Reaktionen. Wandreaktoren können daher auf einem höheren Temperaturniveau als bei polytroper Fahrweise betrieben werden. Daraus resultierend können in katalytischen Wandreaktoren höhere Raum-Zeit-Ausbeuten erzielt werden. Durch den guten Wärmetransport können auch besonders aktive Katalysatormaterialien eingesetzt werden, deren Wärmeentwicklung in konventionellen Reaktoren nicht kontrolliert werden kann.

Weiterhin ist es grundsätzlich möglich, dass in den jeweiligen Reaktoren das jeweilige Katalysatormaterial (Dehydrier-Katalysatormaterial, erstes Oxidations-Katalysatormaterial, zweites Oxidations-Katalysatormaterial oder das eine Direktoxidation katalysierende Katalysatormaterial) sowohl auf den Oberflächen des Reaktors (im Falle eines Rohrbündelreaktors auf den Innenflächen der Reaktionsrohe und im Falle eines Wandreaktors in üblicherweise auf den plattenförmigen Wandelementen) als auch auf den Oberflächen des offenzelligen, metallischen oder keramischen Schaumkörpers aufgebracht ist.

Weiterhin ist es gemäß einer besonderen Variante des erfindungsgemäßen Verfahrens bevorzugt, dass in mindestens einem der Reaktoren ausgewählt aus dem Dehydrier-Reaktor, dem ersten Oxidations-Reaktor, dem zweiten Oxidations-Reaktor oder demjenigen Reaktor, in dem eine Direktoxidation durchgeführt wird, vorzugsweise jedoch in dem ersten Oxidations-Reaktor, dem zweiten Oxidations-Reaktor oder dem ersten und dem zweiten Oxidations-Reaktor mindestens zwei voneinander trennbare, mit dem Katalysatormaterial belegte metallische oder keramische Schaumkörper bzw. aus dem Katalysatormaterial geformte Schaumkörper vorhanden sind. Dabei können die Schaumkörper beispielsweise in Form von Füllkörpern, welche etwa die Gestalt von Kugeln oder Ringen aufweisen können, vorliegen, welche in ihren Größenverhältnissen vorzugsweise so ausgelegt sind, dass eine möglichst dichte Packung erreicht werden kann. Solche Füllkörper sind vorzugsweise dadurch gekennzeichnet, dass sie einen Durchmesser aufweisen, der höchstens 50 %, besonders bevorzugt höchstens 40 % und am meisten bevorzugt höchstens 30 %, vorzugsweise jedoch mindestens 5 % und noch mehr bevorzugt mindestens 10 % des Durchmessers des Reaktors (im Falle eines Rohrbündelreaktors des Durchmessers der jeweiligen Reaktionsrohre und im Falle eines Wandreaktors des Abstandes zwischen zwei benachbarten Platten) beträgt. Neben einer Füllkörperform können die Schaumkörper auch in Form von Blöcken, beispielsweise in Form von Zylindern, gegebenenfalls auch Hohlzylindern, eingesetzt werden. Solche Blöcke sind vorzugsweise dadurch gekennzeichnet, dass sie einen Durchmesser aufweisen, der mindestens 50 %, besonders bevorzugt mindestens 75 %, noch mehr bevorzugt mindestens 90 % und am meisten bevorzugt mindestens 95 % des Durchmessers des Reaktors (im Falle eines Rohrbündelreaktors des Durchmessers der jeweiligen Reaktionsrohre und im Falle eines Wandreaktors des Abstandes zwischen zwei benachbarten Platten) beträgt.

Die Anzahl der in den Reaktoren vorhandenen, voneinander trennbaren Schaumkörper hängt von der Form dieser Schaumkörper ab. Gemäß einer ersten besonderen Ausführungsform des erfindungsgemäßen Verfahrens, bei der die Schaumkörper als Füllkörper in Form einer Festbettschüttung in den Reaktor bzw. die Reaktionsrohre des Rohrbündelreaktors eingebracht werden, werden vorzugsweise 500 bis 20.000, besonders bevorzugt 1.000 bis 15.000 und am meisten bevorzugt 2.000 bis 10.000 voneinander trennbare, vorzugsweise mit Katalysatormaterial belegte oder aus Katalysatormaterial geformte Schaumkörper pro Reaktor eingesetzt. Gemäß einer zweiten besonderen Ausführungsform des erfindungsgemäßen Verfahrens, bei der die Schaumkörper nicht in Form einer Festbettschüttung, sonderen in Form von Schaumkörper-Blöcken in den Reaktor bzw. die Reaktionsrohre des Rohrbündelreaktors eingebracht werden, werden vorzugsweise 1 bis 500, besonders bevorzugt 2 bis 100 und am meisten bevorzugt 3 bis 50 voneinander trennbare, vorzugsweise mit Katalysatormaterial belegte oder aus Katalysatormaterial geformte Schaumkörper-Blöcke pro Reaktor eingesetzt.

Im Zusammenhang mit der vorstehend beschriebenen besonderen Variante des erfindungsgemäßen Verfahrens kann es sich weiterhin als vorteilhaft erweisen, wenn sich die mindestens zwei voneinander trennbaren, mit dem jeweiligen Katalysatormaterial belegten oder aus dem Katalysatormaterial geformten Schaumkörper eines Reaktors durch ihre Katalysator-Aktivität, angegeben in der Aktivität pro Volumeneinheit des Katalysators, unterscheiden. Dabei kann es sich insbesondere im Dehydrier-Reaktor, im ersten Oxidations-Reaktor, im zweiten Oxidations-Reaktor, im Dehydrier-Reaktor und im ersten Oxidations-Reaktor, im Dehydrier-Reaktor und im zweiten Oxidations-Reaktor, im ersten Oxidations-Reaktor und im zweiten Oxidations-Reaktor, im Dehydrier-Reaktor, im ersten und im zweiten Oxidations-Reaktor oder in demjenigen Reaktor, in dem eine Direktoxidation durchgeführt wird, als vorteilhaft erweisen, wenn die mindestens zwei voneinander trennbaren, mit dem Katalysatormaterial belegten oder aus dem Katalysatormaterial geformten Schaumkörper derart innerhalb des Reaktors angeordnet sind, dass die katalytische Aktivität pro Volumenelement des Reaktors in der Richtung vom Edukt-Einlass (also vom Einlass des gesättigten Kohlenwasserstoffs im Falle des Dehydrier-Reaktors oder des zur Direktoxidation eingesetzten Reaktors, vom Einlass des ungesättigten Kohlenwasserstoffs im Falle des ersten Oxidations-Reaktors oder vom Einlass des ungesättigten Aldehyds im Falle des zweiten Oxidations-Reaktors) zum Produkt-Auslass (also zum Auslass für den ungesättigten Kohlenwasserstoff im Falle des Dehydrier-Reaktors oder für die ungesättigte Carbonsäure im Falle eines zur Direktoxidation eingesetzten Reaktors, zum Auslass für den ungesättigten Aldehyd im Falle des ersten Oxidations-Reaktors oder zum Auslass der ungesättigten Carbonsäure im Falle des zweiten Oxidations-Reaktors) zunimmt.

Die Variation in der Katalysator-Aktivität der jeweiligen Schaumkörper innerhalb eines Reaktors kann dabei beispielsweise durch folgende Maßnahmen, die gegebenenfalls auch miteinander kombiniert werden können, erzielt werden: Anpassung der Katalysator-Dichte und/oder der Katalysator-Masse in den einzelnen Schaumkörpern (beispielsweise durch den Einsatz von Schaumkörpern mit unterschiedlicher Poren pro Meter Zahl (ppi-Zahl)), Wahl von Katalysatormaterialien unterschiedlicher Aktivität, unterschiedliche Dotierung des Katalysatormaterials mit Inertstoffen, Auftragen des Katalysatormaterials in unterschiedlicher Dichte und/oder Masse (in dem im Verfahrensschritt A1) Suspensionen mit unterschiedlicher Konzentration hinsichtlich des Katalysatormaterials hergestellt werden oder beispielsweise die Verfahrenschritte A1) bis A3), insbesondere auch die Verfahrensschritte A1) bis A4) mehrfach hintereinander durchgeführt werden).

Weiterhin kann es erfindungsgemäß vorteilhaft sein, in den jeweiligen Reaktoren mit dem Katalysatormaterial beschichtete oder aus Katalysatormaterial geformte, offenzellige Schaumkörper oder aber offenzellige, aus Katalysatormaterial geformte Schaumkörper mit herkömmlichen Festbettschüttungen (also Katalysatoren, welche keine offenzelligen Schaumkörper beinhalten) zu kombinieren. Solche herkömmlichen Festbettschüttungen können beispielsweise aus zu Füllkörpern, beispielsweise Kugeln oder Ringen, geformten Katalysatormaterialien oder aus nicht katalytisch aktiven Füllkörpern, welche mit dem Katalysatormaterial beschichtet im sind, bestehen. Eine solche Kombination kann innerhalb eines Reaktionsrohres eines Rohrbündelreaktors oder innerhalb eines Plattenzwischenraumes eines Wandreaktors realisiert werden oder beispielsweise auch dadurch, dass zwei Reaktoren, beispielsweise zwei Rohrbündelreaktoren oder zwei Wandreaktoren hintereinander angeordnet werden, wobei im ersten Reaktor beispielsweise der mit dem Katalysatormaterial belegte oder der aus dem Katalysatormaterial geformte, offenzellige Schaumkörper und im zweiten Reaktor eine herkömmliche Kätalysator-Festbettschüttung vorgesehen ist.

In diesem Zusammenhang ist es insbesondere bevorzugt, dass im Falle eines Reaktors mit der Länge L, wobei der Punkt L₀ der Edukt-Einlass und der Punkt Lₘₐₓ der Produkt-Auslass des Reaktors ist,
- der mit dem Katalysatormaterial beschichtete, offenzellige Schaumkörper oder aber der bevorzugt, aus Katalysatormaterial geformte Schaumkörper in einen Bereich des Reaktors (im Falle eines Rohrbündelreaktors in einen Bereich eines jeden Reaktionsrohres und im Falle eines Wandreaktors in einen Bereich zwischen jeweils zwei benachbarten Platten) eingebracht wird, der von L₀ bis höchstens 0,9 ×Lₘₐₓ, besonders bevorzugt von L₀ bis höchstens 0,8 ×Lₘₐₓ und am meisten bevorzugt von L₀ bis höchstens 0,7 ×Lₘₐₓ reicht, und
- die herkömmlichen Festbettschüttungen in einen Bereich eines jeden Reaktionsrohres (im Falle eines Rohrbündelreaktors in einen Bereich eines jeden Reaktionsrohres und im Falle eines Wandreaktors in einen Bereich zwischen jeweils zwei benachbarten Platten) eingebracht wird, der von mindestens 0,5 × Lₘₐₓ bis Lₘₐₓ, besonders bevorzugt mindestens 0,6 × Lₘₐₓ bis Lₘₐₓ und am meisten bevorzugt von mindestens 0,7 × Lₘₐₓ bis Lₘₐₓ reicht.

Die Schaumkörper werden demnach besonders bevorzugt im Bereich der vorderen 2/3 des Reaktors eingebracht, gefolgt von einer herkömmlichen Festbettschüttung.

Einen Beitrag zur Lösung der eingangs genannten Aufgaben leistet weiterhin ein Verfahren zur Herstellung aufgereinigter, ungesättigter Carbonsäuren, vorzugsweise zur Herstellung von Acrylsäure oder Methacrylsäure, beinhaltend die Verfahrensschritte:
(I) Herstellung eines eine ungesättigte Carbonsäure, vorzugsweise Acrylsäure oder Methacrylsäure, beinhaltenden Gasgemisches durch das vorstehend beschriebene, erfindungsgemäße Verfahren zur Herstellung von ungesättigten Carbonsäuren;
(II) Absorption der ungesättigte Carbonsäure, vorzugsweise der Acrylsäure oder der Methacrylsäure, in einem Lösungsmittel unter Erhalt einer eine ungesättigte Carbonsäure, vorzugsweise Acrylsäure oder Methacrylsäure, beinhaltenden Lösung;
(III) Abtrennung der ungesättigten Carbonsäure, vorzugsweise der Acrylsäure oder der Methacrylsäure, aus der die ungesättigte Carbonsäure, vorzugsweise die Acrylsäure oder die Methacrylsäure beinhaltenden Lösung mittels Destillation, Extraktion, Kristallisation oder einer Kombination dieser Abtrennverfahren.

Als Lösungsmittel im Verfahrensschritt II) kommen Wasser oder eine organische Verbindung mit einem Siedepunkt in einem Bereich von 50 bis 250°C, vorzugsweise in einem Bereich von 70 bis 180°C und darüber hinaus bevorzugt in einem Bereich von 105 bis 150°C oder Wasser und diese organische Verbindung in Betracht. Als derartige organische Verbindung kommen insbesondere Aromaten und darüber hinaus bevorzugt alkylierte Aromaten in Betracht. Üblicherweise wird das als Quenchmittel wirkende Lösungsmittel mit dem Monomergas in einer geeigneten Kolonne, vorzugsweise im Gegenstrom, in Kontakt gebracht.

Es ist in diesem Zusammenhang insbesondere bevorzugt, dass die im Verfahrensschritt II) erhaltene Lösung (Quenchphase) die ungesättigte Carbonsäure, vorzugsweise die Acrylsäure oder die Methacrylsäure, in einer Menge in einem Bereich von 30 bis 90 Gew.-%, vorzugsweise in einem Bereich von 35 bis 85 Gew.-%, und darüber hinaus bevorzugt in einem Bereich von 45 bis 75 Gew.-%, jeweils bezogen auf die Quenchphase, beinhaltet. Weiterhin ist es bevorzugt, dass die Aufarbeitung der Quenchphase bei Temperaturen unterhalb des Siedepunktes der ungesättigten Carbonsäure erfolgt. Im Falle von Acrylsäure besteht eine hierfür geeignete Maßnahme darin, dass ein entsprechend kaltes Quenchmittels mit einer Temperatur von unter 40°C eingesetzt wird. Die so temperierte Quenchphase kann dann einer Extraktion oder Kristallisation oder beides zur Aufarbeitung gemäß Verfahrensschritt III) zugeführt werden, wobei die Temperaturen im Falle der Acrylsäure vorzugsweise in einem Bereich von -40 bis 40°C, vorzugsweise in einem Bereich von -20 bis 39°C und besonders bevorzugt in einem Bereich von - 10 bis 35°C liegen.

Im Verfahrensschritt III) wird sodann die Quenchphase aufgearbeitet. Für den Fall, dass das Quenchmittel zu mindestens 50 Gew.-%, vorzugsweise mindestens 70 Gew.-%, aus Wasser besteht, ist es bevorzugt, dass das mit der ungesättigten Carbonsäure, beispielsweise mit Acrylsäure befrachtete wässrige Quenchmittel in einem weiteren Schritt mit einem Trennmittel, das vorzugsweise nicht mit Wasser gut löslich ist, aufgearbeitet wird. Die carbonsäurereichste Phase wird entweder einer Destillation oder einer Kristallisation oder beidem, vorzugsweise zunächst einer Kristallisation, unterzogen. Die Kristallisation kann sowohl als Schicht- als auch als Suspensionskristallisation durchgeführt werden. Geeignete Schichtkristallisationsvorrichtungen sind kommerziell von der Sulzer AG zu erhalten. Geeignete Suspensionskristallisationsverfahren bedienen sich in der Regel eines Kristallerzeugers gefolgt von einer Waschkolonne. Derartige Vorrichtungen und Verfahren sind von der Niro Prozesstechnologie BV kommerziell zu erhalten. Als Extraktions/Trennmittel kommt im Fall von Acrylsäure als ungesättigte Carbonsäure insbesondere eine aromatische Verbindung, darüber hinaus bevorzugt ein Alkylaromat und weiterhin bevorzugt Toluol in Betracht. Sollte eine organische Verbindung als Trennmittel eingesetzt werden, so kann diese mit Acrylsäure befrachtete organische Verbindung ebenfalls sowohl einer Destillation als auch einer Kristallisation oder einer Kombination von beiden unterzogen werden. Eine hierfür geeignete Kristallisation ist in EP-A-1 015 410 offenbart.

Einen Beitrag zur Lösung der eingangs genannten Aufgaben leistet weiterhin ein Verfahren zur Herstellung eines auf ungesättigten Carbonsäuren, vorzugsweise auf Acrylsäure oder Methacrylsäure basierenden Polymers, beinhaltend die Verfahrensschritte:
(α1) Herstellung eines aufgereinigten ungesättigten Kohlenwasserstoffs, vorzugsweise von Acrylsäure oder Methacrylsäure, durch das vorstehend beschriebene Verfahren;
(α2) radikalische Polymerisation des ungesättigten Kohlenwasserstoffs, vorzugsweise der Acrylsäure oder der Methacrylsäure.

Diese radikalische Polymerisation gemäß Verfahrensschritt (α2) erfolgt vorzugsweise in Form einer wässrigen Lösungspolymerisation oder einer Emulsions- oder Suspensionspolymerisation, bei der der ungesättigte Kohlenwasserstoff in vorzugsweise teilneutralisierter Form in Gegenwart von Vernetzter unter Erhalt eines Polymergels vorzugsweise radikalisch polymerisiert wird. Das so erhaltene Polymergel wird anschließend, sofern es durch Lösungspolymerisation erhalten wurde, zerkleinert und sodann getrocknet. Im Falle eines Einsatzes von Acrylsäure als ungesättigte Carbonsäure können auf diese Weise wasserabsorbierende Polymergebilde erhalten werden, die sich insbesondere als Superabsorber zum Einsatz in Hygieneartikeln, wie beispielsweise Windeln, eignen. Weitere Einzelheiten zur Herstellung derartiger wasserabsorbierender Polymergebilde durch radikalische Polymerisation von Acrylsäure können unter anderem dem 3. Kapitel in "Modern Superabsorbent Polymer Technology", F. L. Buchholz und A. T. Graham (Herausgeber), Wiley-VCH, New York, 1998, entnommen werden.

Einen Beitrag zur Lösung der eingangs genannten Aufgaben leistet weiterhin eine Vorrichtung der Herstellung von aufgereinigten, ungesättigten Carbonsäuren, vorzugsweise von Acrylsäure oder Methacrylsäure, umfassend als Vorrichtungsbestandteile:
(β1) gegebenenfalls einen Reaktor zur Direktoxidation gesättigter Kohlenwasserstoffe, aufweisend ein Katalysatormaterial, welches die Umsetzung gesättigter Kohlenwasserstoffe zu ungesättigten Carbonsäuren katalysiert;
(β2) gegebenenfalls einen Dehydrier-Reaktor aufweisend ein Dehydrier-Katalysatormaterial;
(β3) gegebenenfalls einen gegebenenfalls mit dem Dehydrier-Reaktor (β2) fluidleitend verbundenen ersten Oxidations-Reaktor aufweisend ein erstes Oxidations-Katalysatormaterial;
(β4) gegebenenfalls einen mit dem ersten Oxidations-Reaktor (β3) fluidleitend in Verbindung stehenden zweiten Oxidationsreaktor aufweisend ein zweites Oxidations-Katalysatormaterial;
(β5) eine mit dem zweiten Oxidations-Reaktor Reaktor (β1) oder (β3) fluidleitend in Verbindung stehende Quenchvorrichtung;
(β6) eine mit der Quenchvorrichtung (β5) fluidleitend in Verbindung stehende Aufreinigungseinheit;
wobei mindestens einer der Reaktoren ausgewählt aus dem Reaktor zur Direktoxidation (β1), dem Dehydrier-Reaktor (β2), dem ersten Oxidations-Reaktor (β3) und dem zweiten Oxidations-Reaktor (β4), vorzugsweise der erste Oxidationsreaktor (β3), der zweite Oxidations-Reaktor (β4) oder der erste und der zweite Oxidations-Reaktor (β3) und (β4), mindestens einen offenzelligen, metallischen oder keramischen Schaumkörper beinhaltet.

Als Dehydrier- bzw. erste oder zweite Oxidations-Katalysatormaterialien, als Katalysatormaterialien für eine Direktoxidation sowie als Dehydrier- bzw. erste oder zweite Oxidations-Reaktoren und als Reaktor für eine Direktoxidation kommen dabei grundsätzlich diejenigen Katalysatormaterialien bzw. Reaktoren in Betracht, die bereits eingangs im Zusammenhang mit dem erfindungsgemäßen Verfahren zur Herstellung einer ungesättigten Carbonsäure als bevorzugte Katalysatormaterialien bzw. Reaktoren genannt wurden. Auch als bevorzugt offenzellige, metallische oder keramische Schaumkörper kommen grundsätzlich ebenfalls diejenigen bevorzugt offenzelligen Schaumkörper in Betracht, die eingangs im Zusammenhang mit dem erfindungsgemäßen Verfahren zur Herstellung einer ungesättigten Carbonsäure als bevorzugte bevorzugt offenzellige Schaumkörper genannt wurden.

Auch im Zusammenhang mit der erfindungsgemäßen Vorrichtung ist es besonders bevorzugt, dass mindestens ein Teil der Oberfläche des offenzelligen, metallischen oder keramischen Schaumkörpers mit dem Dehydrier-Katalysatormaterial, mit dem ersten oder zweiten Oxidations-Katalysatormaterial oder dem Katalysatormaterial für eine Direktoxidation belegt ist. Vorzugsweise ist dieser mit dem Katalysatormaterial belegte bevorzugt offenzellige Schaumkörper erhältlich durch das eingangs beschriebene Beschichtungsverfahren umfassend die Verfahrensschritte A1) bis A5).

Gemäß einer besonderen Ausführungsform der erfindungsgemäßen Vorrichtung handelt es sich bei mindestens einer der Reaktoren ausgewählt aus Reaktor zur Direktoxidation (β1), dem Dehydrier-Reaktor (β2), dem ersten Oxidations-Reaktor (β3) und dem zweiten Oxidations-Reaktor (β4), vorzugsweise jedoch bei dem ersten Oxidationsreaktor (β3), dem zweiten Oxidations-Reaktor (β4) oder dem ersten und dem zweiten Oxidations-Reaktor (β3) und (β4) um einen Rohrbündelreaktor umfassend mindestens zwei Reaktionsrohre ist, wobei das Dehydrier-Katalysatormaterial, das erste oder zweite Oxidations-Katalysatormaterial bzw. das Katalysatormaterial zur Direktoxidation und der offenzellige Schaumkörper bzw. der mit dem Dehydrier-Katalysatormaterial, dem ersten oder dem zweiten Oxidations-Katalysatormaterial oder dem Katalysatormaterial zur Direktoxidation belegte offenzellige Schaumkörper im Inneren der Reaktionsrohre lokalisiert ist/sind.

Auch kann es im Zusammenhang mit der erfindungsgemäßen Vorrichtung vorteilhaft sein, wenn mindestens einer der Reaktoren ausgewählt aus dem Reaktor zur Direktoxidation (β1), dem Dehydrier-Reaktor (β2), dem ersten Oxidations-Reaktor (β3) und dem zweiten Oxidations-Reaktor (β4), vorzugsweise jedoch der erste Oxidationsreaktor (β3), der zweite Oxidations-Reaktor (β4) oder der erste und der zweite Oxidations-Reaktor (β3) und (β4) mindestens zwei voneinander trennbare, mit dem Katalysatormaterial belegte offenzellige Schaumkörper aufweist, wobei sich mindestens zwei Schaumkörper eines Reaktors, wie bereits im Zusammenhang mit dem erfindungsgemäßen Verfahren zur Herstellung von ungesättigten Aldehyden oder ungesättigten Carbonsäuren, durch ihre Katalysator-Aktivität, angegeben in der Aktivität pro Volumeneinheit des Katalysators, unterscheiden, wobei diese unterschiedliche Katalysator-Aktivät der voneinander trennbaren Schaumkörper vorzugsweise durch die im Zusammenhang mit dem erfindungsgemäßen Verfahren zur Herstellung von ungesättigten Aldehyden oder ungesättigten Carbonsäuren beschriebenen Maßnahmen realisiert werden kann. In diesem Zusammenhang kann es weiterhin vorteilhaft sein, wenn die mindestens zwei voneinander trennbaren, mit dem Katalysatormaterial belegten Schaumkörper derart innerhalb der Reaktoren angeordnet sind, dass die katalytische Aktivität pro Volumenelement in der Richtung vom Edukt-Einlass (also vom Einlass des gesättigten Kohlenwasserstoffs im Falle des Dehydrier-Reaktors (β2) oder des zur Direktoxidation eingesetzten Reaktors (β1), vom Einlass des ungesättigten Kohlenwasserstoffs im Falle des ersten Oxidations-Reaktors (β3) oder vom Einlass des ungesättigten Aldehyds im Falle des zweiten Oxidations-Reaktors (β4)) zum Produkt-Auslass (also zum Auslass für den ungesättigten Kohlenwasserstoff im Falle des Dehydrier-Reaktors (β2) oder für die ungesättigte Carbonsäure im Falle eines zur Direktoxidation eingesetzten Reaktors (β1), zum Auslass für den ungesättigten Aldehyd im Falle des ersten Oxidations-Reaktors (β3) oder zum Auslass der ungesättigten Carbonsäure im Falle des zweiten Oxidations-Reaktors(β4)) zunimmt.

Gemäß einer besonderen Ausführungsform der erfindungsgemäßen Vorrichtung beinhaltet diese zusätzlich eine mit der Aufreinigungseinheit (β6) fluidleitend in Verbindung stehende Polymerisationseinheit (β7).

Einen Beitrag zur Lösung der eingangs genannten Aufgaben leistet auch die Verwendung der vorstehend beschriebenen Vorrichtung in dem erfindungsgemäßen Verfahren zur Herstellung von ungesättigten Carbonsäuren, insbesondere zur Herstellung von Acrylsäure oder Methacrylsäure. Einen Beitrag zur Lösung der eingangs genannten Aufgaben leistet insbesondere die Verwendung der vorstehend beschrieben, zusätzlich die Polymerisationseinheit (β7) beinhaltenden Vorrichtung in dem vorstehend beschriebenen, erfindungsgemäßen Verfahren zur Herstellung eines auf ungesättigten Carbonsäuren, vorzugsweise auf Acrylsäure oder Methacrylsäure, basierenden Polymers.

Die Erfindung wird nun anhand nicht limitierender Figuren und Beispiele näher erläutert.

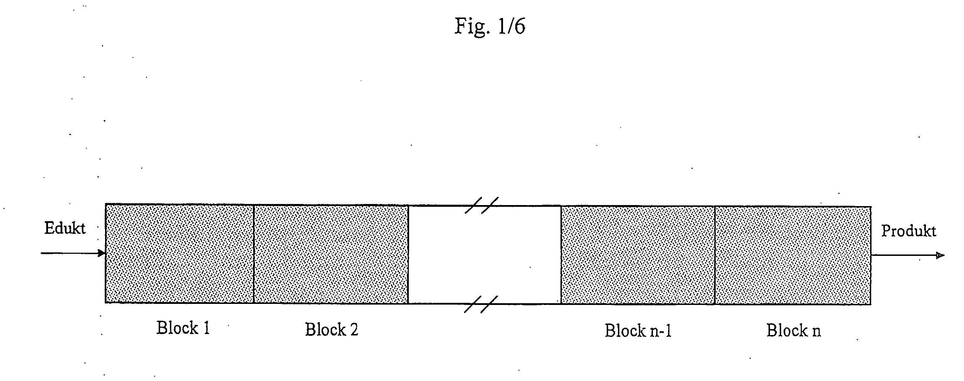

Es zeigt die Figur 1 beispielhaft eine erfindungsgemäße Anordnung von mit Katalysatormaterial belegten Schaumkörpern in Form von voneinander trennbaren Blöcken (Blöcke 1 bis n, wobei nur die Blöcke 1, 2, n-1 und n gezeigt sind und n einen Wert von bis zu 500 annehmen kann) innerhalb eines Reaktors. Gezeigt ist ein einzelnes Reaktionsrohr, welches beispielsweise Bestandteil eines Rohrbündelreaktors sein kann. In Figur 1 weisen die einzelnen Blöcke 1 bis n im Wesentlichen die gleiche katalytische Aktivität pro Volumenelement des Reaktors auf.

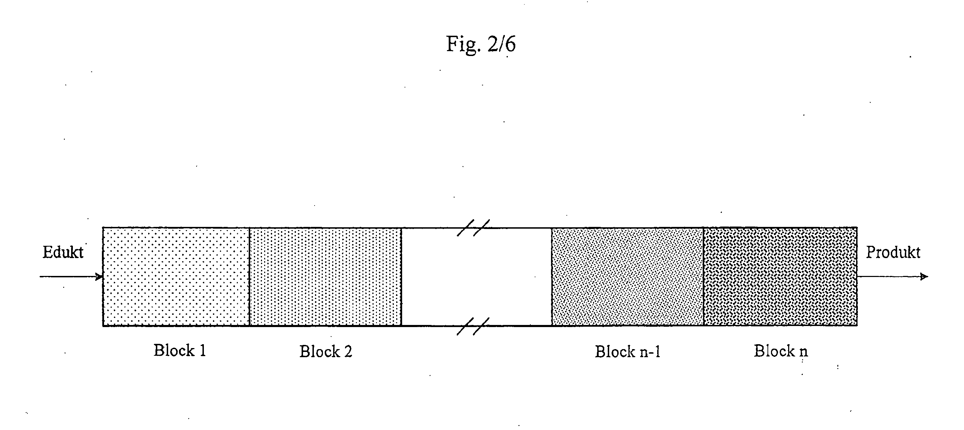

Es zeigt die Figur 2 beispielhaft ebenfalls eine erfindungsgemäße Anordnung von mit Katalysatormaterial belegten Schaumkörpern in Form von voneinander trennbaren Blöcken (Blöcke 1 bis n, wobei wiederum wobei nur die Blöcke 1, 2, n-1 und n gezeigt sind und n einen Wert von bis zu 500 annehmen kann) innerhalb eines Reaktors. Im Gegensatz zur Figur 1 nimmt die katalytische Aktivität pro Volumenelement des Reaktors in der Richtung vom Edukt-Einlass zum Produkt-Auslass zu (angedeutet durch die zunehmende Punktdichte innerhalb der Blöcke 1 bis n in Figur 2).

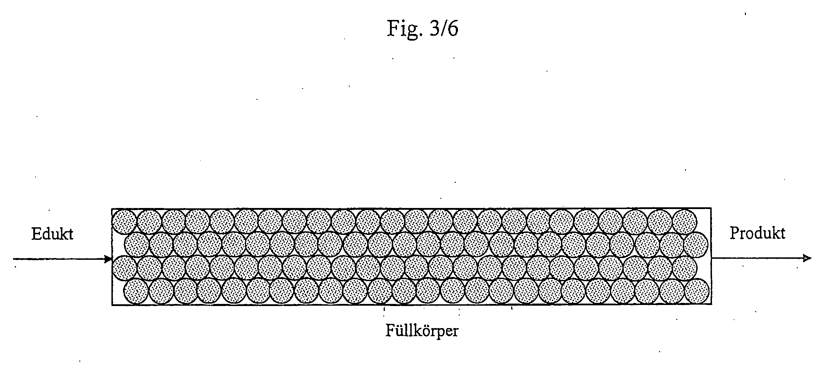

Es zeigt die Figur 3 beispielhaft eine erfindungsgemäße Anordnung von mit Katalysatormaterial belegten Schaumkörpern in Form einer Füllkörperschüttung aus kugelförmigen, mit dem Katalysatormaterial belegten Schaumkörpern innerhalb eines Reaktors, wobei die einzelnen Schaumkörper im Wesentlichen die gleiche katalytische Aktivität pro Volumenelement aufweisen.

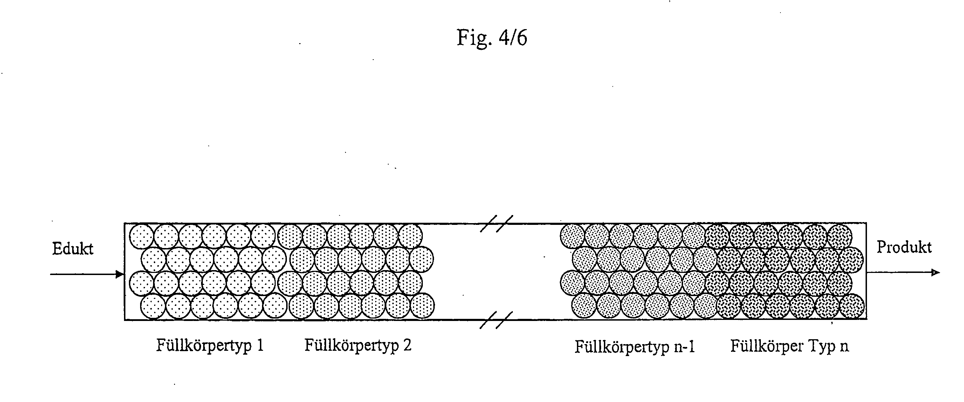

Es zeigt die Figur 4 beispielhaft eine weitere, erfindungsgemäße Anordnung von mit Katalysatormaterial belegten Schaumkörpern in Form einer Füllkörperschüttung aus kugelförmigen, mit dem Katalysatormaterial belegten Schaumkörpern innerhalb eines Reaktors, wobei gemäß dieser Anordnung Schaumkörper mit unterschiedlicher katalytische Aktivität pro Volumenelement (Füllkörpertyp 1 bis n, wobei nur die Füllkörpertypen 1, 2, n-1 und n gezeigt sind und n auch hier einen Wert von bis zu 500 annehmen kann) eingesetzt werden. Gemäß dieser Ausführungsform nimmt die katalytische Aktivität pro Volumenelement des Reaktors in der Richtung vom Edukt-Einlass zum Produkt-Auslass zu (angedeutet durch die zunehmende Punktdichte innerhalb der einzelnen, kugelförmigen Füllkörper in Figur 4).

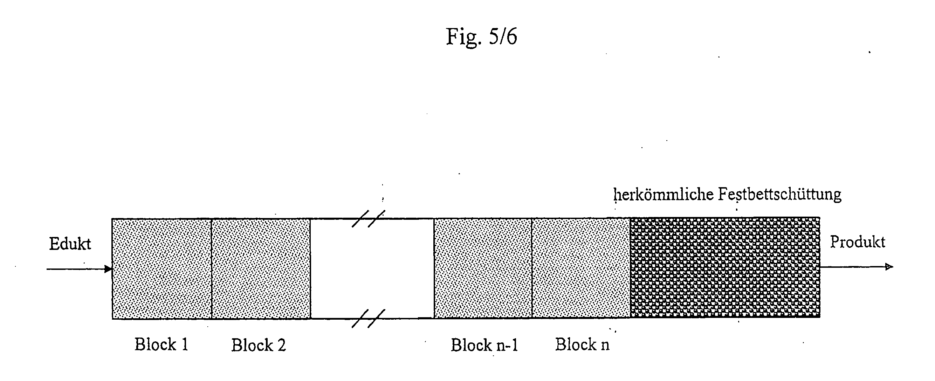

Es zeigt die Figur 5 eine besondere Ausführungsform des erfindungsgemäßen Verfahrens, bei der innerhalb eines Reaktors, beispielsweise innerhalb eines Reaktionsrohres eines Rohrbündelreaktors, sowohl mit Katalysatormaterial belegte Schaumkörper (in der Figur 5 in Form der Schaumkörper-Blöcke 1 bis n, wobei wiederum wobei nur die Blöcke 1, 2, n-1 und n gezeigt sind und n einen Wert von bis zu 500 annehmen kann) als auch eine herkömmlichen Festbettschüttung eingesetzt werden. Der Schaumkörper ist dabei im Bereich der vorderen zwei Drittel des Reaktionsrohres angeordnet, während die herkömmliche Festbettschüttung im hinteren Drittel des Reaktors lokalisiert ist. Auch bei dieser Ausführungsform kann die katalytische Aktivität pro Volumenelement des Reaktors in dem mit dem Schaumkörper belegten Bereich in der Richtung vom Edukt-Einlass zum Produkt-Auslass zunehmen (in Figur 5 nicht gezeigt).

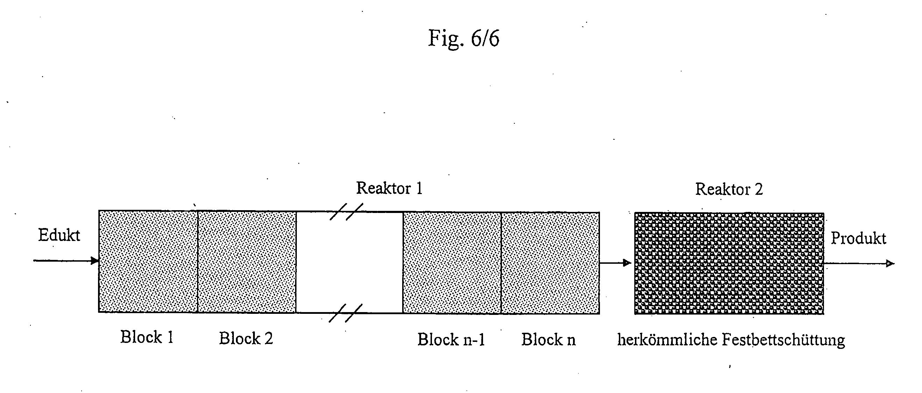

Es zeigt die Figur 6 eine besondere Ausgestaltung der in Figur 5 beschriebenen Vorgehensweise, wobei mit Katalysatormaterial belegte Schaumkörper (in der Figur 6 in Form der Schaumkörper-Blöcke 1 bis n, wobei wiederum wobei nur die Blöcke 1, 2, n-1 und n gezeigt sind und n einen Wert von bis zu 500 annehmen kann) und eine herkömmliche Festbettschüttung nicht innerhalb des gleichen Reaktors, sondern in Form von zwei hintereinander liegenden Reaktoren miteinander kombiniert werden. Auch bei dieser Ausführungsform kann die katalytische Aktivität pro Volumenelement des Reaktors in dem mit dem Schaumkörper belegten Bereich (Reaktor 1) in der Richtung vom Edukt-Einlass zum Produkt-Auslass zunehmen (in Figur 6 nicht gezeigt).

### BEISPIEL

4.81 g eines Katalysator-Pulvers (Mo-V-W-Cu) werden zusammen mit 1.79 g Bindemittel (Acti-Gel) eingewogen. Dazu werden 54 ml Wasser gegeben. Die Komponenten werden gerührt, bis sich eine homogene Suspension ergibt. Die Herstellung des Katalysator-Pulvers erfolgte nach der in Kunert et al., Appl. Catal., A 269 (2004), Seiten 53-61 beschriebenen Vorgehensweise.

Ein gereinigter Edelstahl-Schaum-Träger (63.2 mm × 20.0 mm; 787,4 Poren pro Meter (20 ppi); Masse: 13.41g) wird in die erhaltene Suspension eingetaucht (dipcoating), so dass dieser vollständig mit der Suspension bedeckt ist. Die Dauer des Beschichtungsvorganges beträgt 5 Minuten. Suspension und Träger werden während dieser Zeit stets gegeneinander bewegt.

Nach dem Beschichtungsvorgang wird der Schwamm von überschüssiger Restsuspension durch ein Heißluftgebläse befreit und 180 min bei 120°C im Luftstrom getrocknet. Anschließend erfolgt ein 5-stündiges Kalzinieren bei 390°C im Kalzinierofen.

Der auf diese Weise erhaltene, mit dem Katalysatormaterial belegte Schaumkörper wird in einem Verfahren gemäß Beispiel 1 der WO-A-03/051809 in der ersten und der zweiten Oxidationsstufe zur Herstellung von Acrylsäure aus Propen eingesetzt.

Dabei zeigt sich, dass im Vergleich zu einer herkömmlichen Festbettschüttung die Temperaturdifferenz zwischen dem Wärmeübertragungsmittel und dem Katalysatormaterial deutlich geringer war.

## Patentansprüche

1. Ein Verfahren zur Herstellung von ungesättigten Aldehyden oder ungesättigten Carbonsäuren durch heterogene, katalytische Gasphasenoxidation von ungesättigten oder gesättigten Kohlenwasserstoffen, beinhaltend die Verfahrensschritte:
i) Bereitstellung eines Gasgemisches mindestens beinhaltend einen gesättigten Kohlenwasserstoff und katalytische Gasphasendehydrierung des mindestens einen gesättigten Kohlenwasserstoffs unter Erhalt eines einen ungesättigten Kohlenwasserstoff enthaltenen Gasgemisches in einem ein Dehydrier-Katalysatormaterial aufweisenden Dehydrier-Reaktor; oder
ii) Bereitstellung eines Gasgemisches mindestens beinhaltend Sauerstoff und mindestens einen ungesättigten Kohlenwasserstoff;
iii) katalytische Gasphasenoxidation des in Verfahrensschritt i) erhaltenen oder des in Verfahrensschritt ii) bereitgestellten ungesättigten Kohlenwasserstoffs unter Erhalt eines einen ungesättigten Aldehyd enthaltenen Gasgemisches in einem ersten, ein erstes Oxidations-Katalysatormaterial aufweisenden Oxidations-Reaktor;
iv) gegebenenfalls katalytische Gasphasenoxidation des im Verfahrensschritt iii) erhaltenen, ungesättigten Aldehyds unter Erhalt eines eine ungesättigte Carbonsäure enthaltenen Gasgemisches in einem zweiten, ein zweites Oxidations-Katalysatormaterial aufweisenden Oxidations-Reaktor;
wobei mindestens einer der Reaktoren ausgewählt aus dem Dehydrier-Reaktor, dem ersten Oxidations-Reaktor und dem zweiten Oxidations-Reaktor mindestens einen metallischen oder keramischen offenzelligen Schaumkörper beinhaltet und der metallische oder keramische Schaumkörper durch eine Zellweite in einem Bereich von 39,4 bis 1574,8 Poren pro Meter (1 bis 40 ppi) gekennzeichnet ist.

2. Das Verfahren nach Anspruch 1, wobei das Verfahren die Verfahrensschritte ii), iii) und iv) umfasst, der ungesättigte Kohlenwasserstoff Propen, der ungesättigte Aldehyd Acrolein und die ungesättigte Carbonsäure Acrylsäure ist.

3. Das Verfahren nach Anspruch 1, wobei das Verfahren die Verfahrensschritte ii), iii) und iv) umfasst, der ungesättigte Kohlenwasserstoff Isobuten, der ungesättigte Aldehyd Methacrolein und die ungesättigte Carbonsäure Methacrylsäure ist.

4. Das Verfahren nach Anspruch 1, wobei das Verfahren die Verfahrensschritte i), ii), iii) und iv) umfasst, der gesättigte Kohlenwasserstoff Propan, der ungesättigte Kohlenwasserstoff Propen, der ungesättigte Aldehyd Acrolein und die ungesättigte Carbonsäure Acrylsäure ist.

5. Das Verfahren nach Anspruch 1, wobei das Verfahren die Verfahrensschritte i), ii), iii) und iv) umfasst, der gesättigte Kohlenwasserstoff Isobutan, der ungesättigte Kohlenwasserstoff Isobuten, der ungesättigte Aldehyd Methacrolein und die ungesättigte Carbonsäure Methacrylsäure ist.

6. Das Verfahren nach einem der vorhergehenden Ansprüche, wobei Belastung an ungesättigtem Kohlenwasserstoff bei mindestens 150/h liegt.

7. Das Verfahren nach einem der vorhergehenden Ansprüche, wobei der Dehydrier-Reaktor den Schaumkörper beinhaltet.

8. Das Verfahren nach einem der Ansprüche 1 bis 7, wobei der erste Oxidations-Reaktor den Schaumkörper beinhaltet.

9. Das Verfahren nach einem der Ansprüche 1 bis 7, wobei der zweite Oxidations-Reaktor den Schaumkörper beinhaltet.

10. Das Verfahren nach einem der vorhergehenden Ansprüche, wobei der erste und der zweite Oxidations-Reaktor den Schaumkörper beinhalten.

11. Das Verfahren nach einem der vorhergehenden Ansprüche, wobei mindestens ein Teil der Oberfläche des offenzelligen Schaumkörpers, der eine Zellweite in einem Bereich von 39,4 bis 1574,8 Poren pro Meter (1 bis 40 ppi) aufweist und mit dem Dehydrier-Katalysatormaterial, dem ersten Oxidations-Katalysatormaterial, dem zweiten Oxidations-Katalysatormaterial oder einem Katalysatormaterial, welches die Direktoxidation eines gesättigten Kohlenwasserstoffs zu einer ungesättigten Carbonsäure katalysiert, belegt ist.

12. Das Verfahren nach Anspruch 11, wobei der mit dem Katalysatormaterial belegte offenzellige Schaumkörper der eine Zellweite in einem Bereich von 39,4 bis 1574,8 Poren pro Meter (1 bis 40 ppi) aufweist und erhältlich ist durch ein Verfahren beinhaltend die Verfahrenschritte;
A1) Herstellung einer Suspension aus dem Katalysatormaterial, einem Lösungsmittel oder einem Lösungsmittelgemisch sowie gegebenenfalls weiteren Additiven;
A2) In Kontakt bringen mindestens eines Teils der Oberfläche des Schaumkörpers mit der Suspension;
A3) gegebenenfalls Austragen eines Überschusses an Suspension;
A4) gegebenenfalls Trocknen des Schaumkörpers;
A5) Kalzinieren des Katalysatormaterials.

13. Das Verfahren nach einem der vorhergehenden Ansprüche, wobei der Schaumkörper selbst aus einem Material mit katalytischer Aktivität geformt ist.

14. Das Verfahren nach einem der vorhergehenden Ansprüche, wobei mindestens einer der Reaktoren ausgewählt aus dem Dehydrier-Reaktor, dem ersten Oxidations-Reaktor, dem zweiten Oxidations-Reaktor oder einem Reaktor zur Direktoxidation gesättigter Kohlenwasserstoff zu ungesättigten Carbonsäuren ein Rohrbündelreaktor umfassend mindestens zwei Reaktionsrohre ist, wobei der Dehydrier-Katalysator, der erste Oxidations-Katalysator, der zweite Oxidations-Katalysator bzw. das Katalysatormaterial, welches die Direktoxidation eines gesättigten Kohlenwasserstoffs zu einer ungesättigten Carbonsäure katalysiert, und der Schaumkörper bzw. der mit dem Dehydrier-Katalysator, dem ersten Oxidations-Katalysator, dem zweiten Oxidations-Katalysator bzw. dem Katalysatormaterial, welches die Direktoxidation eines gesättigten Kohlenwasserstoffs zu einer ungesättigten Carbonsäure katalysiert, belegte oder aus diesem Katalysatormaterial geformte Schaumkörper im Inneren der Reaktionsrohre lokalisiert ist/sind.

15. Das Verfahren nach Anspruch 14, wobei mindestens zwei Rohrbündelreaktoren parallel zueinander betrieben werden.

16. Das Verfahren nach einem der Ansprüche 1 bis 13, wobei mindestens einer der Reaktoren ausgewählt aus dem Dehydrier-Reaktor, dem ersten Oxidations-Reaktor, dem zweiten Oxidations-Reaktor oder einem Reaktor zur Direktoxidation gesättigter Kohlenwasserstoff zu ungesättigten Carbonsäuren ein Wandreaktor ist, wobei der Dehydrier-Katalysator, der erste Oxidations-Katalysator, der zweite Oxidations-Katalysator bzw. das Katalysatormaterial, welches die Direktoxidation eines gesättigten Kohlenwasserstoffs zu einer ungesättigten Carbonsäure katalysiert, und der Schaumkörper bzw. der mit dem Dehydrier-Katalysator, dem ersten Oxidations-Katalysator, dem zweiten Oxidations-Katalysator bzw. dem Katalysatormaterial, welches die Direktoxidation eines gesättigten Kohlenwasserstoffs zu einer ungesättigten Carbonsäure katalysiert, belegte oder aus diesem Katalysatormaterial geformte Schaumkörper in dem Reaktionsraum zwischen zwei benachbarten Platten lokalisiert ist.

17. Das Verfahren nach einem der vorhergehenden Ansprüche, wobei in mindestens einem der Reaktoren ausgewählt aus dem Dehydrier-Reaktor, dem ersten Oxidations-Reaktor, dem zweiten Oxidations-Reaktor oder einem Reaktor zur Direktoxidation gesättigter Kohlenwasserstoff zu ungesättigten Carbonsäuren mindestens zwei voneinander trennbare, mit dem Katalysatormaterial belegte oder aus diesem Katalysatormaterial geformte Schaumkörper vorhanden sind.

18. Das Verfahren nach Anspruch 17, wobei die mindestens zwei voneinander trennbaren, mit dem Katalysatormaterial belegten oder aus dem Katalysatormaterial geformten Schaumkörper als Füllkörper in einer Festbettschüttung oder als Schaumkörper-Blöcke vorliegen.

19. Das Verfahren nach Anspruch 17 oder 18, wobei sich mindestens zwei der voneinander trennbaren Schaumkörper eines Reaktors durch ihre katalytische Aktivität unterscheiden.

20. Das Verfahren nach Anspruch 19, wobei der Dehydrier-Reaktor, der erste Oxidations-Reaktor, der zweite Oxidations-Reaktor oder der Reaktor zur Direktoxidation die mindestens zwei voneinander trennbaren, mit dem Katalysatormaterial belegten oder aus dem Katalysatormaterial geformten Schaumkörper beinhaltet/beinhalten und wobei die mindestens zwei voneinander trennbaren, mit dem Katalysatormaterial belegten oder aus dem Katalysatormaterial geformten Schaumkörper derart innerhalb des Dehydrier-Reaktors, des ersten Oxidations-Reaktors, des zweiten Oxidations-Reaktors oder des Reaktors zur Direktoxidation angeordnet sind, dass die katalytische Aktivität pro Volumenelement in der Richtung vom Edukt-Einlass zum Produkt-Auslass zunimmt.

21. Ein Verfahren zur Herstellung aufgereinigter, ungesättigter Carbonsäuren, beinhaltend die Verfahrensschritte:
(I) Herstellung eines eine ungesättigte Carbonsäure beinhaltenden Gasgemisches durch ein Verfahren nach einem der Ansprüche 1 bis 20;
(II) Absorption der ungesättigten Carbonsäure in einem Lösungsmittel unter Erhalt einer eine ungesättigte Carbonsäure beinhaltenden Lösung;
(III) Abtrennung der ungesättigten Carbonsäure aus der die ungesättigte Carbonsäure beinhaltenden Lösung mittels Destillation, Extraktion, Kristallisation oder einer Kombination dieser Abtrennverfahren.

22. Ein Verfahren zur Herstellung eines auf einer ungesättigten Carbonsäure basierenden Polymers, beinhaltend die Verfahrensschritte:
(α1) Herstellung einer aufgereinigten, ungesättigten Carbonsäure durch ein Verfahren nach Anspruch 21;
(α2) radikalische Polymerisation der ungesättigten Carbonsäure.

## Claims

1. A process for preparing unsaturated aldehydes or unsaturated carboxylic acids by heterogeneous catalytic gas phase oxidation of unsaturated or saturated hydrocarbons, comprising the process steps of:
i) providing a gas mixture at least comprising a saturated hydrocarbon and catalytically dehydrogenating the at least one saturated hydrocarbon in the gas phase to obtain a gas mixture comprising an unsaturated hydrocarbon in a dehydrogenation reactor having a dehydrogenation catalyst material; or
ii) providing a gas mixture at least comprising oxygen and at least one unsaturated hydrocarbon;
iii)catalytically oxidizing the unsaturated hydrocarbon obtained in process step i) or provided in process step ii) in the gas phase to obtain a gas mixture comprising an unsaturated aldehyde in a first oxidation reactor having a first oxidation catalyst material;
iv) optionally catalytically oxidizing the unsaturated aldehyde obtained in process step iii) in the gas phase to obtain a gas mixture comprising an unsaturated carboxylic acid in a second oxidation reactor having a second oxidation catalyst material;
wherein at least one of the reactors selected from the dehydrogenation reactor, the first oxidation reactor and the second oxidation reactor comprises at least one metallic or ceramic open-cell foam body and the metallic or ceramic foam body is **characterized by** a cell width within a range from 39.4 to 1574.8 pores per metre (1 to 40 ppi).

2. The process according to Claim 1, wherein the process comprises process steps ii), iii) and iv), and the unsaturated hydrocarbon is propene, the unsaturated aldehyde acrolein and the unsaturated carboxylic acid acrylic acid.

3. The process according to Claim 1, wherein the process comprises process steps ii), iii) and iv), and the unsaturated hydrocarbon is isobutene, the unsaturated aldehyde methacrolein and the unsaturated carboxylic acid methacrylic acid.

4. The process according to Claim 1, wherein the process comprises process steps i), ii), iii) and iv), and the saturated hydrocarbon is propane, the unsaturated hydrocarbon propene, the unsaturated aldehyde acrolein and the unsaturated carboxylic acid acrylic acid.

5. The process according to Claim 1, wherein the process comprises process steps i), ii), iii) and iv), and the saturated hydrocarbon is isobutane, the unsaturated hydrocarbon isobutene, the unsaturated aldehyde methacrolein and the unsaturated carboxylic acid methacrylic acid.

6. The process according to one of the preceding claims, wherein the space velocity of unsaturated hydrocarbon is at least 150/h.

7. The process according to one of the preceding claims, wherein the dehydrogenation reactor comprises the foam body.

8. The process according to one of the Claims 1 to 7, wherein the first oxidation reactor comprises the foam body.

9. The process according to one of Claims 1 to 7, wherein the second oxidation reactor comprises the foam body.

10. The process according to one of the preceding claims, wherein the first and the second oxidation reactors comprise the foam body.

11. The process according to one of the preceding claims, wherein at least part of the surface of the open-cell foam body which has a cell width within a range from 39.4 to 1574.8 pores per metre (1 to 40 ppi) is coated with the dehydrogenation catalyst material, the first oxidation catalyst material, the second oxidation catalyst material or a catalyst material which catalyses the direct oxidation of a saturated hydrocarbon to an unsaturated carboxylic acid.

12. The process according to Claim 11, wherein the open-cell foam body which has been coated with the catalyst material and has a cell width within a range from 39.4 to 1574.8 pores per metre (1 to 40 ppi) is obtainable by a process comprising the process steps of:
A1) preparing a suspension composed of the catalyst material, a solvent or a solvent mixture and optionally further additives;
A2) contacting at least part of the surface of the foam body with the suspension;
A3) if appropriate discharging an excess of suspension;
A4) if appropriate drying the foam body;
A5) calcining the catalyst material.

13. The process according to one of the preceding claims, wherein the foam body itself is formed from a material with catalytic activity.

14. The process according to one of the preceding claims, wherein at least one of the reactors selected from the dehydrogenation reactor, the first oxidation reactor, the second oxidation reactor or a reactor for direct oxidation of saturated hydrocarbon to unsaturated carboxylic acids is a tube bundle reactor comprising at least two reaction tubes, and the dehydrogenation catalyst, the first oxidation catalyst, the second oxidation catalyst or the catalyst material which catalyses the direct oxidation of a saturated hydrocarbon to an unsaturated carboxylic acid, and the foam body or the foam body coated with the dehydrogenation catalyst, the first oxidation catalyst, the second oxidation catalyst or the catalyst material which catalyses the direct oxidation of a saturated hydrocarbon to an unsaturated carboxylic acid, or that formed from this catalyst material, is/are localized in the interior of the reaction tubes.

15. The process according to Claim 14, wherein at least two tube bundle reactors are operated in parallel to one another.

16. The process according to one of Claims 1 to 13, wherein at least one of the reactors selected from the dehydrogenation reactor, the first oxidation reactor, the second oxidation reactor or a reactor for direct oxidation of saturated hydrocarbon to unsaturated carboxylic acids is a wall reactor, and the dehydrogenation catalyst, the first oxidation catalyst, the second oxidation catalyst or the catalyst material which catalyses the direct oxidation of a saturated hydrocarbon to an unsaturated carboxylic acid, and the foam body or the foam body coated with the dehydrogenation catalyst, the first oxidation catalyst, the second oxidation catalyst or the catalyst material which catalyses the direct oxidation of a saturated hydrocarbon to an unsaturated carboxylic acid, or that formed from this catalyst material, is localized in the reaction chamber between two adjacent plates.

17. The process according to one of the preceding claims, wherein at least two separable foam bodies coated with the catalyst material or formed from this catalyst material are present in at least one of the reactors selected from the dehydrogenation reactor, the first oxidation reactor, the second oxidation reactor or a reactor for direct oxidation of saturated hydrocarbon to unsaturated carboxylic acids.

18. The process according to Claim 17, wherein the at least two separable foam bodies coated with the catalyst material or formed from the catalyst material are present as random packings in a fixed bed or as foam body blocks.

19. The process according to Claim 17 or 18, wherein at least two of the separable foam bodies of one reactor differ by their catalytic activity.

20. The process according to Claim 19, wherein the dehydrogenation reactor, the first oxidation reactor, the second oxidation reactor or the reactor for direct oxidation comprise(s) the at least two separable foam bodies coated with the catalyst material or formed from the catalyst material, and wherein the at least two separable foam bodies coated with the catalyst material or formed from the catalyst material are arranged within the dehydrogenation reactor, the first oxidation reactor, the second oxidation reactor or the reactor for direct oxidation such that the catalytic activity per volume element increases in the direction from the reactant inlet to the product outlet.

21. A process for preparing purified unsaturated carboxylic acids, comprising the steps of:
(I) preparing a gas mixture comprising an unsaturated carboxylic acid by a process according to one of Claims 1 to 20;
(II) absorbing the unsaturated carboxylic acid in a solvent to obtain a solution comprising an unsaturated carboxylic acid;
(III) removing the unsaturated carboxylic acid from the solution comprising the unsaturated carboxylic acid by means of distillation, extraction, crystallization or a combination of these removal methods.

22. A process for preparing a polymer based on an unsaturated carboxylic acid, comprising the process steps of:
(α1) preparing a purified unsaturated carboxylic acid by a process according to Claim 21;
(α2) free-radically polymerizing the unsaturated carboxylic acid.

## Revendications

1. Procédé pour la préparation d'aldéhydes insaturés ou d'acides carboxyliques insaturés par oxydation hétérogène, catalytique en phase gazeuse d'hydrocarbures insaturés ou saturés, comprenant les étapes de procédé :
i) mise à disposition d'un mélange gazeux comprenant au moins un hydrocarbure saturé et déshydrogénation catalytique en phase gazeuse dudit au moins un hydrocarbure saturé avec obtention d'un mélange gazeux contenant un hydrocarbure insaturé dans un réacteur de déshydrogénation présentant un matériau catalytique de déshydrogénation ; ou
ii) mise à disposition d'un mélange gazeux contenant au moins de l'oxygène et au moins un hydrocarbure insaturé ;
iii) oxydation catalytique en phase gazeuse de l'hydrocarbure insaturé obtenu dans l'étape de procédé i) ou mis à disposition dans l'étape de procédé ii) avec obtention d'un mélange gazeux contenant un aldéhyde insaturé dans un premier réacteur d'oxydation présentant un premier matériau catalytique d'oxydation ;
iv) le cas échéant oxydation catalytique en phase gazeuse de l'aldéhyde insaturé obtenu dans l'étape de procédé iii) avec obtention d'un mélange gazeux contenant un acide carboxylique insaturé dans un deuxième réacteur d'oxydation présentant un deuxième matériau catalytique d'oxydation ;
au moins un des réacteurs choisi parmi le réacteur de déshydrogénation, le premier réacteur d'oxydation et le deuxième réacteur d'oxydation contenant au moins un corps en mousse, métallique ou céramique, à cellules ouvertes et le corps en mousse étant **caractérisé par** une densité de cellules dans une plage de 39,4 à 1574,8 pores par mètre (1 à 40 ppi).

2. Procédé selon la revendication 1, le procédé comprenant les étapes de procédé ii), iii) et iv), l'hydrocarbure insaturé étant le propène, l'aldéhyde insaturé étant l'acroléine et l'acide carboxylique insaturé étant l'acide acrylique.

3. Procédé selon la revendication 1, le procédé comprenant les étapes de procédé ii), iii) et iv), l'hydrocarbure insaturé étant l'isobutène, l'aldéhyde insaturé étant la méthacroléine et l'acide carboxylique insaturé étant l'acide méthacrylique.

4. Procédé selon la revendication 1, le procédé comprenant les étapes de procédé i), ii), iii) et iv), l'hydrocarbure saturé étant le propane, l'hydrocarbure insaturé étant le propène, l'aldéhyde insaturé étant l'acroléine et l'acide carboxylique insaturé étant l'acide acrylique.

5. Procédé selon la revendication 1, le procédé comprenant les étapes de procédé i), ii), iii) et iv), l'hydrocarbure saturé étant l'isobutane, l'hydrocarbure insaturé étant l'isobutène, l'aldéhyde insaturé étant la méthacroléine et l'acide carboxylique insaturé étant l'acide méthacrylique.

6. Procédé selon l'une quelconque des revendications précédentes, la charge en hydrocarbure insaturé se situant à au moins 150/h.

7. Procédé selon l'une quelconque des revendications précédentes, le réacteur de déshydrogénation contenant le corps en mousse.

8. Procédé selon l'une quelconque des revendications 1 à 7, le premier réacteur d'oxydation contenant le corps en mousse.

9. Procédé selon l'une quelconque des revendications 1 à 7, le deuxième réacteur d'oxydation contenant le corps en mousse.

10. Procédé selon l'une quelconque des revendications précédentes, le premier et le deuxième réacteur d'oxydation contenant le corps en mousse.

11. Procédé selon l'une quelconque des revendications précédentes, au moins une partie de la surface du corps en mousse à cellules ouvertes, qui présente une densité de cellules dans une plage de 39,4 à 1574,8 pores par mètre (1 à 40 ppi), étant revêtue par le matériau catalytique de déshydrogénation, le premier matériau catalytique d'oxydation, le deuxième matériau catalytique d'oxydation ou un matériau catalytique qui catalyse l'oxydation directe d'un hydrocarbure saturé en un acide carboxylique insaturé.

12. Procédé selon la revendication 11, le corps en mousse à cellules ouvertes, qui présente une densité de cellules dans une plage de 39,4 à 1574,8 pores par mètre (1 à 40 ppi), revêtu par le matériau catalytique, pouvant être obtenu par un procédé comprenant les étapes de procédé :
A1) préparation d'une suspension du matériau catalytique, d'un solvant ou d'un mélange de solvants ainsi que le cas échéant d'autres additifs ;
A2) mise en contact d'au moins une partie de la surface du corps en mousse avec la suspension ;
A3) le cas échéant, évacuation d'un excès de suspension ;
A4) le cas échéant, séchage du corps en mousse ;
A5) calcination du matériau catalytique.

13. Procédé selon l'une quelconque des revendications précédentes, le corps en mousse lui-même étant formé en un matériau présentant une activité catalytique.

14. Procédé selon l'une quelconque des revendications précédentes, au moins un des réacteurs choisi parmi le réacteur de déshydrogénation, le premier réacteur d'oxydation, le deuxième réacteur d'oxydation ou un réacteur pour l'oxydation directe d'un hydrocarbure saturé en acides carboxyliques insaturés étant un réacteur à faisceau tubulaire comprenant au moins deux tubes de réaction, le catalyseur de déshydrogénation, le premier catalyseur d'oxydation, le deuxième catalyseur d'oxydation ou le matériau catalytique qui catalyse l'oxydation directe d'un hydrocarbure saturé en un acide carboxylique insaturé, et le corps en mousse ou le corps en mousse revêtu par le catalyseur de déshydrogénation, le premier catalyseur d'oxydation, le deuxième catalyseur d'oxydation ou le matériau catalytique qui catalyse l'oxydation directe d'un hydrocarbure saturé en un acide carboxylique insaturé ou le corps en mousse formé en ce matériau catalytique étant situé(s) à l'intérieur des tubes de réaction.

15. Procédé selon la revendication 14, au moins deux réacteurs à faisceau tubulaire étant exploités parallèlement l'un à l'autre.

16. Procédé selon l'une quelconque des revendications 1 à 13, au moins un des réacteurs choisi parmi le réacteur de déshydrogénation, le premier réacteur d'oxydation, le deuxième réacteur d'oxydation ou un réacteur pour l'oxydation directe d'un hydrocarbure saturé en acides carboxyliques insaturés étant un réacteur à parois, le catalyseur de déshydrogénation, le premier catalyseur d'oxydation, le deuxième catalyseur d'oxydation ou le matériau catalytique qui catalyse l'oxydation directe d'un hydrocarbure saturé en un acide carboxylique insaturé, et le corps en mousse ou le corps en mousse revêtu par le catalyseur de déshydrogénation, le premier catalyseur d'oxydation, le deuxième catalyseur d'oxydation ou le matériau catalytique qui catalyse l'oxydation directe d'un hydrocarbure saturé en un acide carboxylique insaturé ou le corps en mousse formé en ce matériau catalytique étant situé dans l'espace de réaction entre deux plaques adjacentes.

17. Procédé selon l'une quelconque des revendications précédentes, au moins deux corps en mousse pouvant être séparés l'un de l'autre, revêtus par le matériau catalytique ou formés en ce matériau catalytique étant présents dans au moins un des réacteurs choisi parmi le réacteur de déshydrogénation, le premier réacteur d'oxydation, le deuxième réacteur d'oxydation ou un réacteur pour l'oxydation directe d'un hydrocarbure saturé en acides carboxyliques insaturés.

18. Procédé selon la revendication 17, lesdits au moins deux corps en mousse pouvant être séparés l'un de l'autre, revêtus par le matériau catalytique ou formés en ce matériau catalytique se trouvant sous forme de corps de remplissage dans un matériau en vrac en lit fixe ou sous forme de blocs de corps en mousse.

19. Procédé selon la revendication 17 ou 18, au moins deux des corps en mousse pouvant être séparés l'un de l'autre d'un réacteur se distinguant par leur activité catalytique.

20. Procédé selon la revendication 19, le réacteur de déshydrogénation, le premier réacteur d'oxydation, le deuxième réacteur d'oxydation ou le réacteur pour l'oxydation directe comprenant lesdits au moins deux corps en mousse pouvant être séparés l'un de l'autre, revêtus du matériau catalytique ou formés en matériau catalytique et lesdits au moins deux corps en mousse pouvant être séparés l'un de l'autre, revêtus par le matériau catalytique ou formés en matériau catalytique étant disposés à l'intérieur du réacteur de déshydrogénation, du premier réacteur d'oxydation, du deuxième réacteur d'oxydation ou du réacteur pour l'oxydation directe de manière telle que l'activité catalytique par élément de volume augmente dans le sens de l'entrée des produits de départ vers la sortie du produit.

21. Procédé pour la préparation d'acides carboxyliques insaturés purifiés, comportant les étapes de procédé :
(I) préparation d'un mélange gazeux contenant un acide carboxylique insaturé par un procédé selon l'une quelconque des revendications 1 à 20 ;
(II) absorption de l'acide carboxylique insaturé dans un solvant avec obtention d'une solution contenant un acide carboxylique insaturé ;
(III) séparation de l'acide carboxylique insaturé de la solution comprenant l'acide carboxylique insaturé par distillation, extraction, cristallisation ou une combinaison de ces procédés de séparation.

22. Procédé pour la préparation d'un polymère basé sur un acide carboxylique insaturé, comprenant les étapes de procédé :
(α1) préparation d'un acide carboxylique insaturé purifié par un procédé selon la revendication 21 ;
(α2) polymérisation radicalaire de l'acide carboxylique insaturé.
